(19) Europäisches Patentamt
European Patent Office
Office européen des brevets

(11) Publication number: **0 115 413**
**A1**

(12) # EUROPEAN PATENT APPLICATION

(21) Application number: 84300386.4

(22) Date of filing: 23.01.84

(51) Int. Cl.³: C 07 D 405/06
A 61 K 31/40

(30) Priority: 24.01.83 PC T/US83/00098
29.08.83 US 527717

(43) Date of publication of application:
08.08.84 Bulletin 84/32

(84) Designated Contracting States:
AT BE CH DE FR GB IT LI LU NL SE

(71) Applicant: SYNTEX (U.S.A.) INC.
3401 Hillview Avenue
Palo Alto, California 94304(US)

(72) Inventor: Muchowski, Joseph Martin
1720 Banff Drive
Sunnyvale California 94087(US)

(72) Inventor: Ackrell, Jack
192 Webster
Palo Alto California 94301(US)

(72) Inventor: Pfister, Jurg Roland
1500 Oak Avenue
Los Altos California 94022(US)

(72) Inventor: Greenhouse, Robert James
Cerrada de Bezares, No. 9
Mexico 11, DF(MX)

(72) Inventor: Li, Tsung-Tee
26440 Anacapa Drive
Los Altos Hills California 94022(US)

(72) Inventor: Kaiser, Hans Peter
Hammerweg 14
CH-8404 Winterthur(CH)

(74) Representative: Armitage, Ian Michael et al,
MEWBURN ELLIS & CO. 2/3 Cursitor Street
London EC4A 1BQ(GB)

(54) +/- 2-(Phenethyl)-5-((3,4-methylenedioxy)-alpha-hydroxybenzyl)-pyrrolidines, pharmaceutical compositions containing them, processes for their production and intermediates therefor.

(57) A compound of the formula

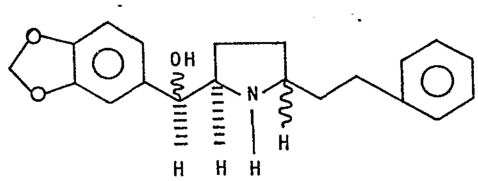

and the pharmaceutically acceptable acid addition salts thereof are potent antihypertensive agents and are therefore useful as cardiovascular system regulators.

EP 0 115 413 A1

±2-[PHENETHYL]-5-[(3,4-METHYLENEDIOXY)-α-HYDROXYBENZYL]PYRROLIDINES, PHARMACEUTICAL COMPOSITIONS CONTAINING THEM, PROCESSES FOR THEIR PRODUCTION AND INTERMEDIATES THEREFOR

This invention concerns ±2-[phenethyl]-5-[(3,4-methylenedioxy)-α-hydroxybenzyl]pyrrolidines which affect the cardiovascular system and which are particularly effective as antihypertensive agents. The invention is directed toward orally active, long lasting cardiovascular regulators of hypertension.

The invention also concerns a method for regulating the cardiovascular system and managing hypertension, and pharmaceutical compositions comprising compounds of this invention as active ingredient.

Various physiological responses result from administering pharmaceuticals which affect the cardiovascular system regulating receptors. These responses may vary from vasodilation, vasoconstriction, tachycardia, bradycardia, positive or negative inotropic effect. Secondary effects such as bronchodilation or bronchoconstriction can also appear.

The physiological response depends on the exact nature of the drug. Therefore, various members of the same general class of compounds may be used in the treatment of cardiac disorders such as hypertension, cardiac arrhythmia, and vasal congestion.

U.S. Patent 4,342,692 and its EPO counterpart disclose a family of compounds which may be interpreted to be similar to the compounds of the current invention.

The present invention is directed toward orally active, long lasting cardiovascular regulators.

The compound of this invention have a strong antihypertensive activity. The antihypertensive activity of the compounds of this invention is best shown by their effect on the systolic blood pressure. The compounds of this invention decrease significantly the systolic blood presure without at the same time increasing heart rates. Moreover, the dosage which is needed to decrease systolic blood pressure is very low and the secondary, often undesirable, side effects are thus avoided. Thus, this invention offers the effective management of hypertension without submitting the treated subject to the undesirable secondary effects which would be unavoidable if large doses are needed.

The invention herein relates to compounds of formula I

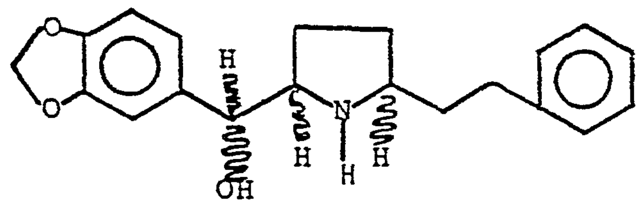

(I)

and their pharmaceutically acceptable acid addition salts, including mixtures of, and individual, stereoisomers, such as cis erythro, cis threo, trans erythro and trans threo isomers of the compounds. These compounds are potent antihypertensive agents and are therefore useful as cardiovascular system regulators.

These compounds of the invention may be prepared in as many as eight possible stereoisomeric forms, and may

exist as these stereochemically pure forms; as racemic mixtures containing both members of one enantomeric pair from the total of four such pairs; or, as mixtures of some or all of the diasteromeric forms. The invention is intended to encompass all of these possibilities.

Separation of diastereomers can, of course, be carried out by any standard separation technique, such as thin layer or high pressure liquid chromatography, or even, fractional crystallization.

Each racemic diastereomer (or enantiomeric pair) may, if desired, be resolved into its optical antipodes by conventional resolution means; for example by separation (e.g. fractional crystallization) of the diastereomeric salts formed by the reaction of these compounds with optically active acids. Exemplary of such optically active acids are the optically active forms of camphor-10-sulfonic acid, 2-bromo-camphor- -sulfonic acid, camphoric acid, menthoxyacetic acid, tartaric acid, malic acid, diacetyltartaric acid, pyrrolidine-5-carboxylic acid and the like. The separated pure diastereomeric salts may then be cleaved by standard means to afford the respective optical isomers of the compounds of Formula I.

Definitions

As used hereinafter:

"Noble metal catalyst" is a catalyst such as platinum on carbon, platinum oxide, palladium on carbon, or rhodium on carbon, but other noble metal catalysts suitable to effect catalytic reductions are also included.

"Protection" or "Protecting group" refer to the protection of phenolic hydroxyl groups. A phenolic hydroxyl group is present in many compounds prepared by the process of this invention. In order to preserve the phenolic hydroxyl group during the catalytic reduction, O-protection is often required for phenols, which react

readily with oxidizing agents, electrophiles, or even with mild alkylating and acylating agents. The protection of phenolic hydroxyl groups can be achieved with any suitable protecting group such as an alkyl ether, for example methyl ether, isopropyl ether, t-butyl ether; alkoxymethyl ether, for example methoxymethyl ether; alkoxyethoxymethyl ether, for example methoxyethoxymethyl ether; cycloalkylmethyl ether, for example cyclopropylmethyl ether; alkyldimethylsilyl ether, for example t-butyldimethylsilyl ether, 9-anthrylmethyl ether, preferably substituted or unsubstituted benzyl ether. [<u>Protective Groups in Organic Synthesis</u>, John Wiley & Sons, New York, pp:87-100(1980); Synthesis, (II): 987 (1982)].

"N-Protection" or "N-Protecting groups" refer to electron withdrawing groups which make pyrrole less aromatic and more susceptible to the reduction. Electron withdrawal achieved through the utilization of N-protection of the nitrogen atom of the pyrrole can be best illustrated by attachment of the acyl N-protecting group, i. e. $R-\underset{O}{C}-$ where R may be aryl, phenyl, substituted phenyl, alkyl of 1-4 carbons with branched alkyl preferred, alkoxy of 1-4 carbons with branched alkoxy preferred Exemplary N-protecting groups for the pyrrole nitrogen atom are alkoxycarbonyl such as methoxycarbonyl, ethoxycarbonyl, propoxycarbonyl, t-butoxycarbonyl and the like, or alkylcarbonyl such as methylcarbonyl, ethylcarbonyl, propylcarbonyl and the like, or alkanoyl such as ethanoyl, propanoyl, butanoyl and the like, or aroyl. These groups are also called $1-R_2$ protecting groups in this application.

"Aroyl" means the radical ZCO- wherein Z is an aromatic group such as, for example, benzoyl or naphthoyl.

"Wt %" (weight percent) used for solids means the weight of one solid relative to the total weight of all

reactants. For example, if 10 wt % of catalyst is given, then 10 g of catalyst are added for 90 g of other reactants.

"Mild reaction conditions" means that the reaction is run at the low temperatures between 10-35°C, preferably ambient and at pressures of 1-5 atmospheres, preferably at atmospheric pressure, in the presence of a suitable polar organic solvent.

"Organic solvent" means liquid organic compound with the power to dissolve solids or liquids at mild reaction conditions. The term is meant to include cyclic and acyclic compounds such as alcohols of 1-4 carbons, lower alkyl esters of alkanoic acids, ethers, cyclic ethers and the like. Examplary solvents are methanol, ethanol, ethyl acetate, tetrahydrofuran, benzene or mixtures thereof.

"Lower alkyl" means a branched or unbranched saturated hydrocarbon chain containing from one to four carbon atoms, such as, for example, methyl, ethyl, n-propyl, iso-propyl, n-butyl, iso-butyl and the like.

"Cycloalkyl" means a saturated monocyclic hydrocarbon of 3-7 carbons without side chains, such as cyclopropane, cyclobutane, cyclopentane, cyclohexane and cycloheptane.

"Alkoxy" means -OR wherein R is lower alkyl as defined hereinabove.

"Alkoxycarbonyl" means -C(O)-OR wherein R is lower alkyl as defined hereinabove.

"Alkylcarbonyl" means -C(O)-R wherein R is lower alkyl as defined hereinabove.

Hereinafter "α-hydroxybenzyl" or "phenylhydroxymethyl" mean compounds of the formula

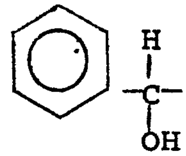

"Strong acid" means an organic or inorganic, water soluble, easily dissociable Bronsted Lowry acid, such as methanesulfonic, trifluoroacetic, hydrochloric, sulfuric, phosphoric acid and the like.

"Strong base" means an inorganic, water soluble base such as sodium hydroxide, sodium carbonate, potassium carbonate, and the like.

"N-acylating" means the formation or introduction of acyl radical $-R-\overset{O}{C}-$ to the N-atom of the pyrrole ring.

Stereochemical Control

The ± 2-[phenethyl]-5-[(3,4-methylenedioxy)-α-hydroxybenzyl] pyrrolidines have three chiral centers. Two chiral centers are at the 2- and 5- positions of the pyrrolidine ring at which the side chains are substituted. The third chiral center is introduced in pyrrolidines where the side chain attached to the 5-position is α-hydroxybenzyl.

Compounds with three chiral centers can be obtained as four diastereoisomeric racemates or as eight optical isomers in total. The nomenclature (±)cis erythro, (±)cis threo, (±)trans erythro and (±)trans threo is used to describe individual diastereoisomers.

Embodiments wherein hydrogens at 2- and 5-positions are on the same side of the plane of the pyrrolidine ring are designated "cis". Embodiments where hydrogens at 2- and 5-position are on opposite sides are "trans."

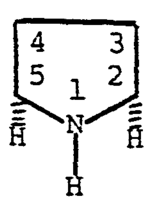

cis-isomer

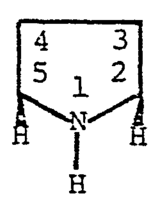

cis-isomer

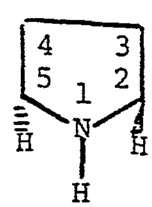

trans-isomer

"Erythro/threo" terminology is used to designate the relationship between the configurations of the group attached to the carbon atom bearing the hydroxyl substituent and of the number 5 carbon of the pyrrolidine ring to which it is attached.

"Erythro" indicates those embodiments wherein the hydrogen of carbon atom 5 of the ring and the hydrogen of the hydroxylated carbon occupy the same side of the molecule.

"Threo" indicates those embodiments where the hydrogen of carbon atom 5 of the ring and the hydrogen of the hydroxylated carbon are on the opposite sides of the molecule. For the numbering system, see below.

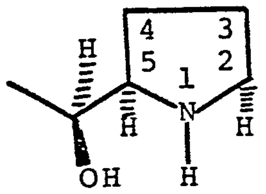

cis-erythro isomer

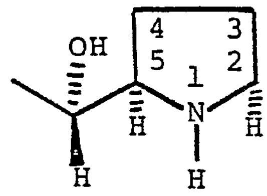

cis-threo isomer

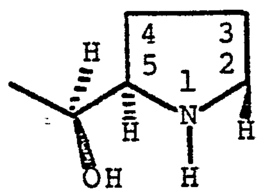

trans-erythro isomer

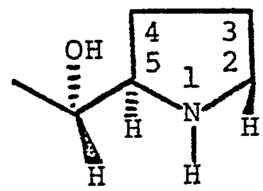

trans-threo isomer

See Stereochemistry of Carbon Compounds, McGraw-Hill, pp. 16-86 (1962); RECUEIL, 83:535, (1964); and Morison and Boyd, Organic Chemistry, 3d Ed., pp. 148-153, (1974).

Numbering on the phenyl rings of the pyrrole or pyrrolidine molecule is illustrated below.

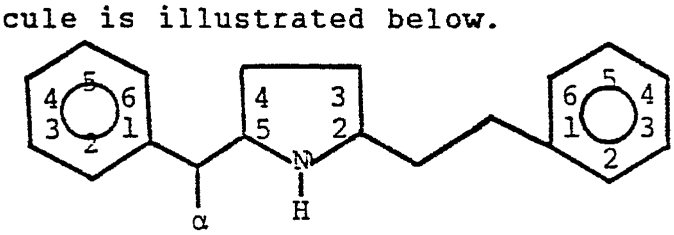

It is to be understood that this invention discloses and encompasses each of the racemates, racemic mixtures, diastereomers and enantiomers.

Presently preferred embodiments of this invention are compounds of the formula

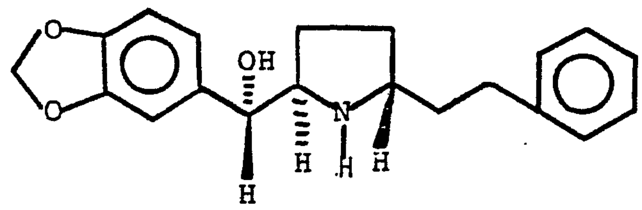

±trans threo

namely, ±trans threo 2-[phenethyl]-5-[(3,4-methylenedioxy)]-α-hydroxybenzyl]pyrrolidine.

More preferred embodiments are compounds of the formula

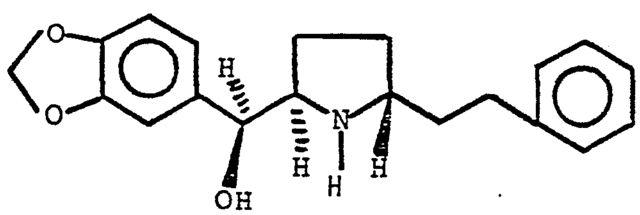

±trans erythro

namely, ±trans erythro 2-[phenethyl]-5-[(3,4-methylenedioxy)]-α-hydroxybenzyl]pyrrolidine.

Other more preferred embodiments are compounds of the formula

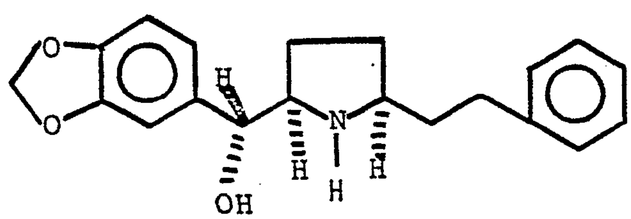

±cis threo

namely, ±cis threo 2-[phenethyl]-5-[(3,4-methylenedioxy)]-α-hydroxybenzyl]pyrrolidine.

Most preferred embodiments are compounds of the formula

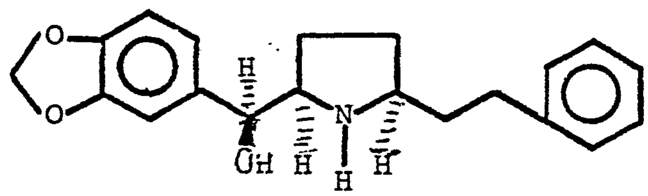

±cis erythro

namely, ±cis erythro 2-[phenethyl]-5-[(3,4-methylenedioxy)]-α-hydroxybenzyl]pyrrolidine.

A compounds of this invention are prepared by the reaction sequence illustrated in Reaction Schemes 1-4.

Reaction Scheme 1 illustrates the preparation of ±cis erythro 2-[phenethyl]-5-[3,4-methylenedioxy)-α-hydroxybenzyl]pyrrolidine. Reaction Scheme 2 illustrates the preparation of ±cis threo 2-[phenethyl]-5-[3,4-methylenedioxy)-α-hydroxybenzyl]-pyrrolidine. Reaction Scheme 3 illustrates preparation of ±trans erythro 2-[phenethyl]-5-[3,4-methylenedioxy) -α-hydroxybenzyl]pyrrolidine. Reaction Scheme 4 illustrates preparation of ±trans threo 2-[phenethyl] -5-[3,4-methylenedioxy)-α-hydroxybenzyl]pyrrolidine.

REACTION SCHEME 1

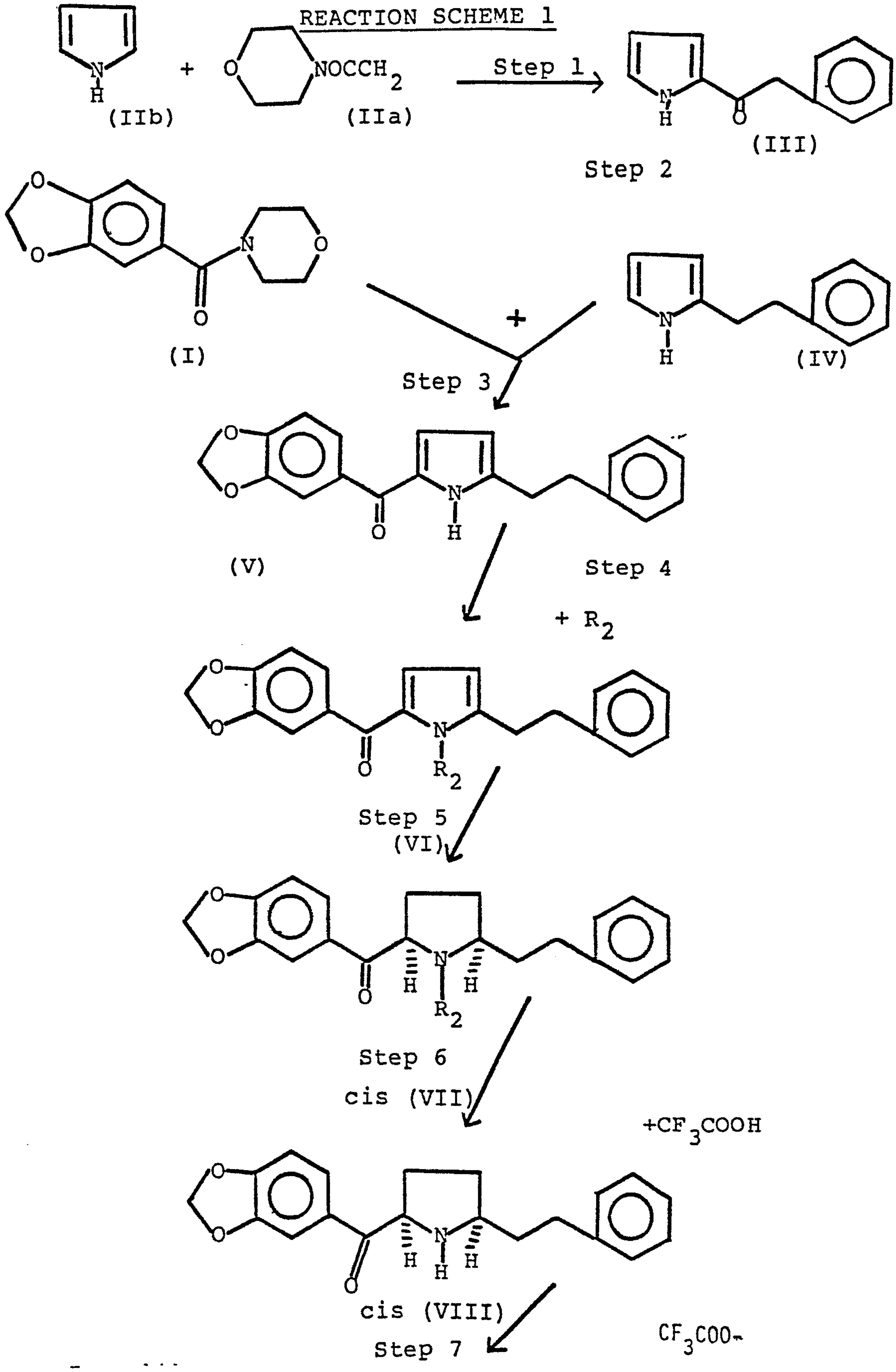


23990

Step 7

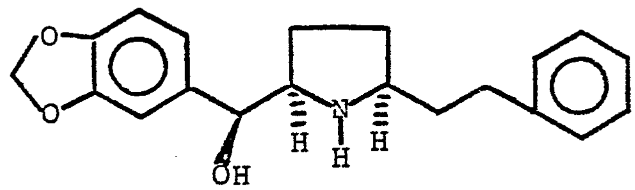

(IX) cis erythro

Step 1. Step 1 describes the preparation of phenylacetylpyrrole (III) from pyrrole (IIb) and phenylacetylmorpholide (IIa).

Pyrrole (IIb) is commercialy available from Aldrich.

Phenylacetylmorpholide (IIa) is prepared by reacting phenylacetic acid with thionyl chloride and with a small amount of dimethylformamide for 10-60 minutes at room temperature. The resulting mixture is reacted with morpholine dissolved in an organic solvent, preferably in dry dichloromethane, to give phenylacetylmorpholide (II).

Phenylacetylmorpholide (IIa) in the presence of an acylating agent, such as acid halides, preferably in phosphorous oxychloride, is reacted under the constant stirring for 3-8 hours, preferably for 6 hours. Pyrrole (IIb) dissolved in a chlorinated hydrocarbon solvent, preferably in anhydrous 1,2-dichloroethane, is added. The reaction mixture is stirred for 12-20 hours, alkalized, and purified by methods known in the art to obtain phenylacetylpyrrole (III).

Step 2. Step 2 describes the conversion of phenylacetylpyrrole (III) to 2-[phenethyl]pyrrole (IV).

Compound (III) is dissolved in an ethereal solvent, preferably anhydrous tetrahydrofuran, and a complex metal hydride, preferably lithium aluminum hydride, is added. The mixture is reacted at reflux temperature for 35-53 hours, preferably 48 hours. Excess of hydride is destroyed with organic solvent and the reaction mixture is purified by methods known in the art to afford 2-[phenethyl]pyrrole (IV).

Step 3. Step 3 describes the preparation of 2-[phenethyl]-5-[(3,4-methylenedioxy)benzoyl]pyrrole (V).

[(3,4-Methylenedioxy)benzoyl]morpholide (I), is prepared from 3,4-methylenedioxybenzoic acid by procedure similar to that of Step 1. (For details, see Example 1.)

[(3,4-Methylenedioxy)benzoyl]morpholide is reacted with an acylating agent, preferably phosphorous oxychloride, at a room temperature for 1-5 hours. Then the compound (IV), dissolved in an organic solvent, preferably in 1,2-dichloroethane, is added and the mixture is stirred for 15-21 hours, preferably for 18 hours. The mixture is purified by methods known in the art to give ±2-[phenethyl]-5-[(3,4-methylenedioxy)-benzoyl]pyrrole (V).

Step 4. Step 4 describes an attachment of the protective group $R_2$ to the N atom of the pyrrole compound (V).

Compound (V) is dissolved in a suspension of an ethereal or dipolar solvent, preferably in dry dimethylformamide, and mixed with sodium hydride. The mixture is heated to 45-60° for 1-3 hours, preferably 2 hours. Suitable N-protecting agent $R_2$, such as aroylchloride, alkanoylchloride, alkylchloroformate, preferably di-t-butylcarbonate, is added and the mixture is stirred at 60-70° for 1-3 hours. After purification and crystallization of methods known in the art, ±1-$R_2$ protected-2-[phenethyl]-5-[3,4-methylenedioxy)-benzoyl]pyrrole, preferably 1-t-butoxycarbonyl-2-[phenethyl]-5-[(3,4-methylenedioxy)benzoyl]pyrrole (VI) is obtained.

Step 5. Step 5 describes a catalytic reduction of 1-t-butoxycarbonyl-2-[phenethyl]-5-[(3,4-methylenedioxy)-benzoyl]pyrrolidine (VII).

Pyrrole (VI) is reduced in the presence of the noble metal catalyst, such as rhodium on carbon, rhodium on

aluminum, platinum on carbon, preferably with platinic oxide, in the solvent or solvent mixture containing lower alcohol, lower alkyl ester or ethereal solvent. The solvent mixture preferred is ethanol-ethyl acetate. Reduction is carried on under the mild reaction conditions, at the room temperature and pressure of 1-3 atmospheres, preferably at atmospheric pressure for 15-24 hours, preferably for 18 hours. The reduced compound is purified and crystallized by the methods known in the art to give ±1-t-butoxycarbonyl-cis-2-[phenethyl]-5-[(3,4-methylenedioxy)benzoyl]pyrrolidine (VII).

Step 6. Step 6 describes the removal of the N-protecting group $R_2$ from the compound (VII).

A solution of 1-t-butoxycarbonyl-cis-2-[phenethyl]-5-[(3,4-methylenedioxy)benzoyl]pyrrolidine or otherwise 1-$R_2$-protected pyrrolidine (VII) in chlorinated hydrocarbon, preferably dry dichloromethane, is added to a strong protic acid, preferably trifluoroacetic acid. The reaction is carried on for 1-3 hours at room temperature. After purification and crystallization by methods known in the art, cis-2-[phenethyl]-5-[(3,4-methylenedioxy)benzoyl]-pyrrolidine trifluoroacetate (VIII) is obtained.

Step 7. Step 7 describes the reduction of cis 2-[phenethyl]-5-[(3,4-methylenedioxy)benzoyl]-pyrrolidine trifluoroacetate (VIII) to ±cis erythro 2-[phenethyl]-5-[(3,4-methylenedioxy)-α-hydroxybenzyl] pyrrolidine (IX).

Compound (VIII) is reduced to compound (IX) with a metal borohydride, preferably sodium borohydride dissolved in lower alcohol, preferably in ethanol at 0° temperature. The mixture is reacted for 0.5-3 hours and the solvent is removed. The aqueous residue is diluted with base such as sodium carbonate and the product is extracted with an organic solvent, preferably with ethyl

acetate. The extract is washed with water, dried over sodium sulfate, purified, and crystallized by the methods known in the art to give ±cis erythro 2-[phenethyl]-5-[(3,4-methylenedioxy)-α-hydroxybenzyl]pyrrolidine (IX).

Reaction Scheme 2 illustrates preparation of cis threo 2-[phenethyl]-5-[(3,4-methylenedioxy)-α-hydroxybenzyl]pyrrolidine.

REACTION SCHEME 2

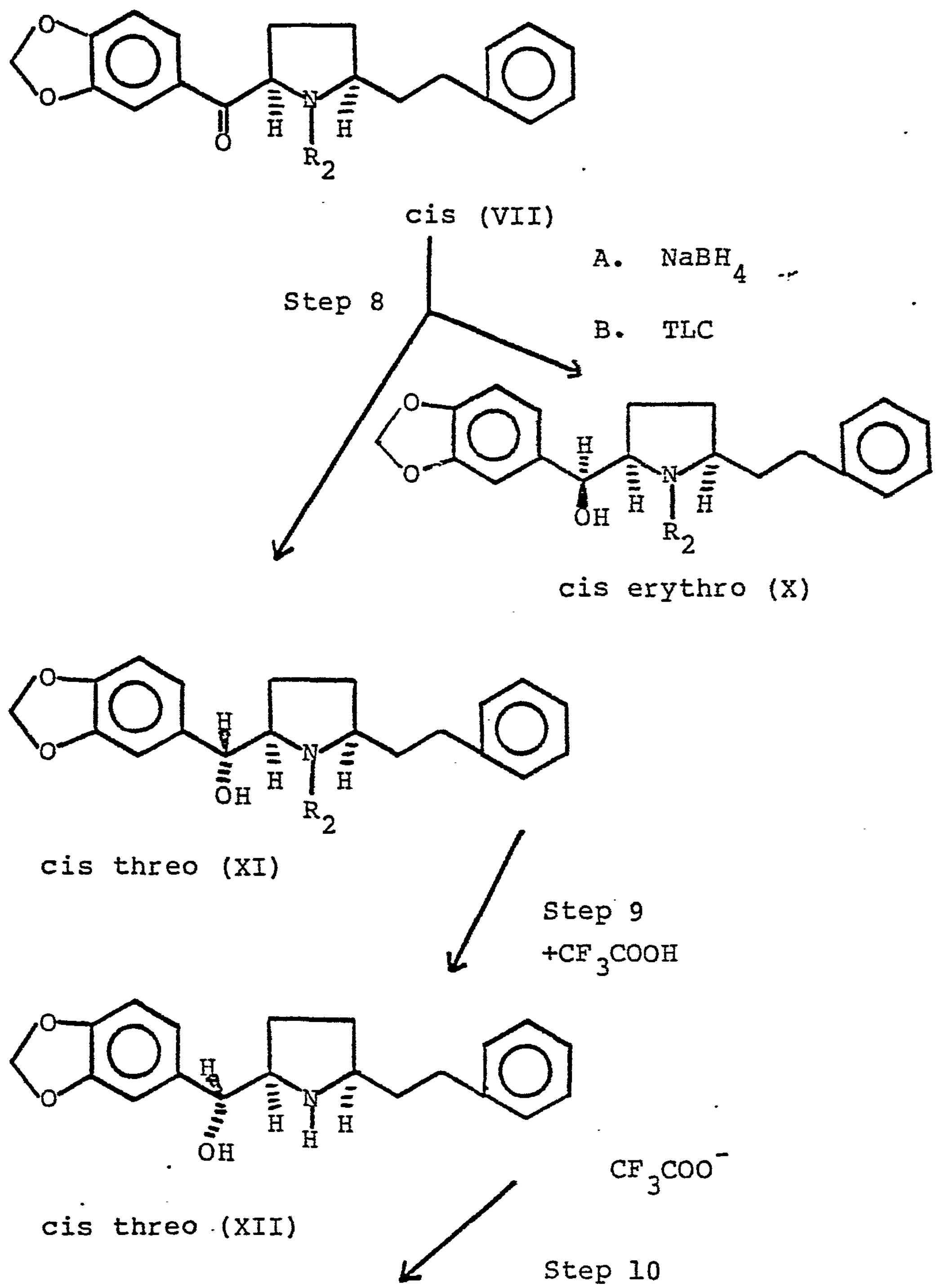


cis (VII)

cis erythro (X)

cis threo (XI)

cis threo (XII)

Step 10

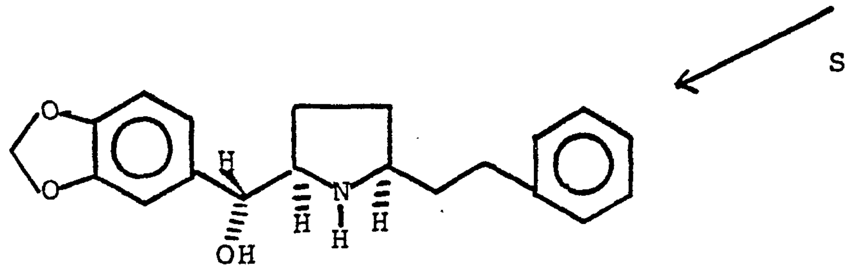

cis threo (XII')

Step 8. Step 8 describes the reduction of keto compound (VII) to the mixture of hydroxy compounds (X) and (XI) and subsequent separation of the obtained mixture into ±cis erythro isomer and ±cis threo isomer of 1-$R_2$-protected-2-[phenethyl]-5-[(3,4-methylenedioxy)-α-hydroxybenzyl]pyrrolidine (X)(XI), respectively.

Step 8A. 1-$R_2$-protected cis 2-[phenethyl]-5-[(3,4-methylenedioxy)benzoyl]pyrrolidine (VII) (see Reaction Scheme 1) is reduced with metal borohydride, preferably with sodium borohydride, in lower alcohol, preferably ethanol or methanol, at -10 to +20°C, for 0.5-50 hours. The product obtained after purification by methods known in the art is the mixture of ±cis erythro and ±cis threo 2-[phenethyl]-5-[(3,4-methylenedioxy)-α-hydroxybenzyl]pyrolidines (X) and (XI), respectively.

Step 8B. Obtained mixture of cis erythro and cis threo isomers (X) and (XI) is separated by thin layer chromatography (TLC), column chromatography, crystallization or any other common separation technique, preferably by TLC, to obtain ±cis erythro 1-t-butoxycarbonyl-2-[phenethyl]-5-[(3,4-methylenedioxy)-α-hydroxybenzyl]pyrrolidine (X) and ±cis threo 1-t-butoxycarbonyl-2-[phenethyl]-5-[(3,4-methylenedioxy)-α-hydroxybenzyl]-pyrrolidine (XI).

Cis erythro compound (X) is then submitted to Step 6 (Reaction Scheme 1) to remove N-protecting group $R_2$, to

result in ±cis erythro 2-[phenethyl]-5-[(3,4-methylenedioxy)-α-hydroxybenzyl]pyrrolidine.

Step 9. Step 9 describes the removal of the N-protecting group from the ±cis threo 1-t-butoxycarbonyl-2-[phenethyl]-5-[(3,4-methylenedioxy)-α-hydroxybenzyl]pyrrolidine.

A solution of cis threo compound (XI) in chlorinated hydrocarbons, preferably dichloromethane, is added to a strong protic acid, such as hydrochloric acid or hydrobromic acid, preferably trifluoroacetic acid. The mixture is reacted for 1-50 hours at -10 to +20°C temperature. The solvent is evaporated and the residue is purified to obtain ±cis threo 2-[(phenethyl]-5-[(3,4-methylenedioxy)-α-hydroxybenzyl]pyrrolidine trifluoroacetate(XII).

Step 10. Step 10 describes conversion of ±cis threo 2-[(phenethyl]-5-[(3,4-methylenedioxy)-α-hydroxybenzyl]-pyrrolidine trifluoroacetate to a free base pyrrolidine (XII').

A trifluoroacetate salt (XII) is dissolved in a water and buffered, preferably with sodium bicarbonate. The resulting free base is extracted into an organic solvent, preferably ethyl acetate and purified by methods known in the art to yield ±cis threo 2-[(phenethyl]-5-[(3,4-methylenedioxy)-α-hydroxybenzyl]pyrrolidine.

REACTION SCHEME 3

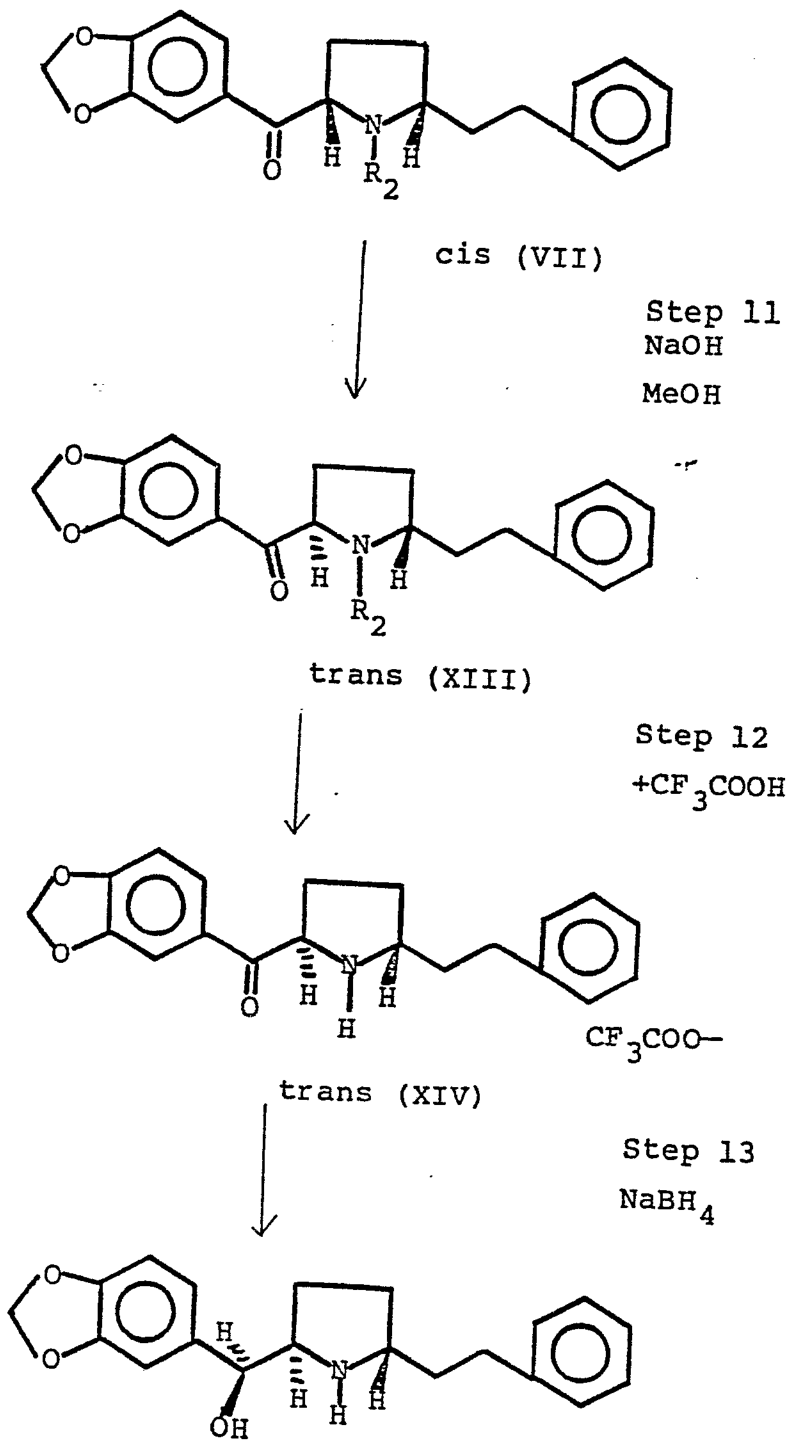


trans erythro (XV)

Reaction Scheme 3 illustrates the preparation of ±trans erythro 2-[phenethyl]-5-[(3,4-methylenedioxy)-α-hydroxybenzyl]pyrrolidines (XVI) and (XVII).

Step 11. Step 11 illustrates the isomerization of N-protected cis 2-[phenethyl]-5-[(3,4-methylenedioxy)-benzoyl]pyrrolidine compound (VII) to the N-protected trans 2-[phenethyl]-5-[(3,4-methylenedioxy)benzoyl]-pyrrolidine compound (XIII).

Isomerization is conducted under strong basic conditions, preferably with alkali hydroxides, such as sodium hydroxide or lithium hydroxide, in a suitable solvent system such as lower alkanol, for example methanol, ethanol, propanol, butanol and such, at the temperature of 20-100°C, for the period of time 20-100 hours. The resulting mixture is rich in the trans isomer N-protected 2-[phenethyl]-5-[(3,4-methylenedioxy)benzoyl]-pyrrolidine (XIII).

Step 12. Step 12 describes the removal of the N-protecting group from ±trans 2-[phenethyl]-5-[(3,4-methylenedioxy)benzoyl]pyrrolidine.

A solution of trans compound (XIII) in chlorinated hydrocarbons, preferably dichloromethane, is added to a strong protic acid, such as hydrochloric acid or hydrobromic acid, preferably trifluoroacetic acid. The mixture is reacted for 1-50 hours at -10 to +20°C temperature. The solvent is evaporated and the residue is purified to obtain ±trans 2-[phenethyl]-5-[(3,4-methylenedioxy)benzoyl]pyrrolidine trifluoroacetate (XIV).

Step 13. Step 13 describes the reduction of trans 2-[phenethyl]-5-[(3,4-methylenedioxy)benzoyl]pyrrolidine compound (XIV) to ±trans erythro 2-[phenethyl]-5-[(3,4-methylenedioxy)-α-hydroxybenzyl]pyrrolidine (XV).

2-[phenethyl]-5-[(3,4-methylenedioxy)benzoyl] pyrrolidine trifluoroacetate (XIV) is reduced with metal

borohydride, preferably with sodium borohydride, in a lower alcohol, preferably ethanol or methanol, at -10 to +20°C. The solvent is removed, the aqueous residue is diluted with the solution of base, preferably saturated sodium carbonate, and the product is extracted to organic halogenated solvent, preferably dichloromethane, to result in ±trans erythro 2-[phenethyl]-5-[(3,4-methylenedioxy)-α-hydroxybenzyl]pyrrolidine compound (XV).

REACTION SCHEME 4

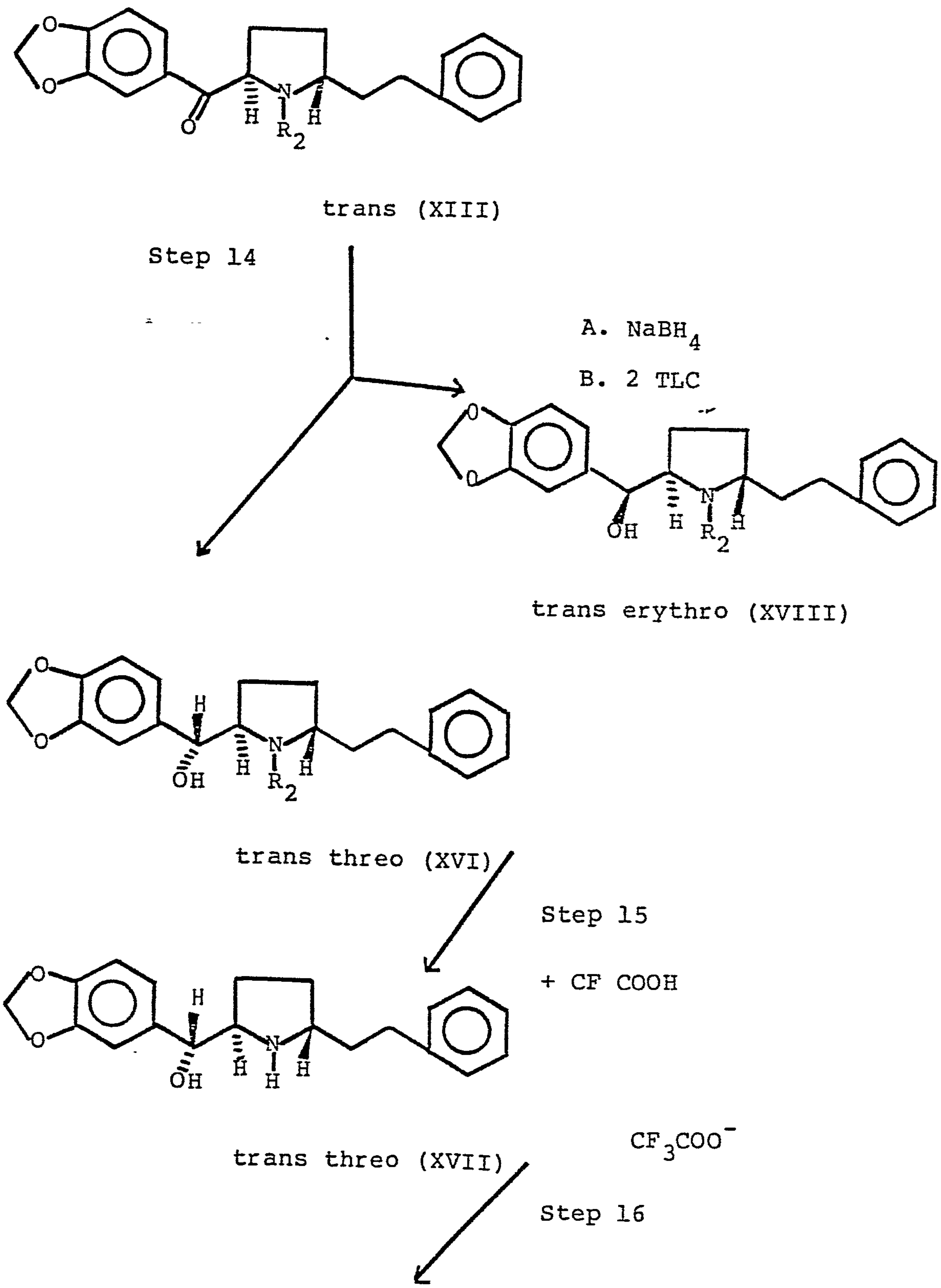


trans (XIII)

trans erythro (XVIII)

trans threo (XVI)

trans threo (XVII)

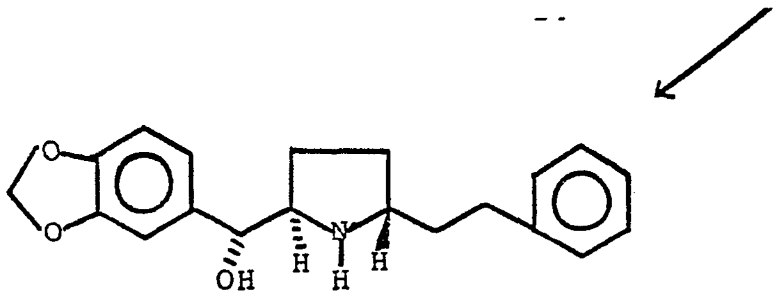

Step 16

trans threo (XVII')

Step 14. Step 14 describes the reduction of N-protected ±trans 2-[phenethyl]-5-[(3,4-methylenedioxy) benzoyl]pyrrolidine compound (XIII) into the N-protected ± trans erythro 2-[phenethyl]-5-[(3,4-methylenedioxy)-α-hydroxybenzyl]pyrrolidine (XV) and ±trans threo 2-[phenethyl]-5-[(3,4-methylenedioxy)-α-hydroxybenzyl]-pyrrolidine (XVI).

Step 14A. The reaction begins with the reduction of compound (XIII) with metal borohydride, preferably with sodium borohydride, in lower alcohol, preferably ethanol or methanol, at -10 to +20°C for 0.5-50 hours. The mixture obtained after purification by methods known in the art consist of ±trans erythro and ±trans threo N-protected 2-[phenethyl]-5-[(3,4-methylenedioxy)-α-hydroxybenzyl]pyrrolidine compounds (XVIII) and (XVI).

Step 14B. The obtained mixture is separated by TLC, column chromatography, crystallization or any other common separation technique, preferably by TLC, to obtain N-protected ±trans erythro 2-[phenethyl]-5-[(3,4-methylenedioxy)hydroxybenzyl]pyrrolidine (XVIII) and ± N-protected trans threo 2-[phenethyl]-5-[(3,4-methylenedioxy)-α-hydroxybenzyl]pyrrolidine (XVI).

N-protected ±trans erythro 2-[phenethyl]-5-[(3,4-methylenedioxy)-α-hydroxybenzyl]pyrrolidine (XVIII) is submitted to the procedure of Step 12 (Reaction Scheme 3).

Step 15. Step 15 describes the removal of the N-protecting group from the ±trans threo 2-[phenethyl]-5-[(3,4-methylenedioxy)-α-hydroxybenzyl]pyrrolidine compound (XVI).

A solution of N-protected trans 2-[phenethyl]-5-[(3,4-methylenedioxy)-α-hydroxybenzyl]pyrrolidine compound (XVI) in chlorinated hydrocarbons, preferably dichloromethane, is added to a strong protic acid, such as hydrochloric acid or hydrobromic acid, preferably trifluoroacetic acid. The mixture is reacted for 1-50 hours at -10 to +20°C temperature. The solvent is evaporated and the residue is purified to obtain the trifluoroacetic acid salt of ±trans threo 2-[phenethyl]-5-[(3,4-methylenedioxy)-α-hydroxybenzyl]pyrrolidine (XX).

Step 16. Step 16 describes conversion of ±trans threo 2-[(phenethyl]-5-[(3,4-methylenedioxy)-α-hydroxybenzyl]pyrrolidine trifluoroacetate to a free base pyrrolidine (XII').

A trifluoroacetate salt (XII) is dissolved in a water and buffered, preferably with sodium bicarbonate. The resulting free base is extracted into an organic solvent, preferably ethyl acetate and purified by methods known in the art to yield ±trans threo 2-[(phenethyl]-5-[(3,4-methylenedioxy)-α-hydroxybenzyl]pyrrolidine.

Isolation, separation, and purification of the desired final compounds and their intermediates from the reaction mixture can be effected by any suitable separation or purification procedure, such as, for example, extraction, filtration, evaporation, distillation, crystallization, thin-layer chromatography, column chromatography, high pressure liquid chromatography, and the like, or by a combination of these procedures. If not otherwise described above, illustrations of suitable isolation, separation and purification procedures can be had by reference to the

Examples herein below. However, other isolation, separation and isolation procedures could, of course, also be used.

In summary, compounds of this invention can be prepared by the following steps:

N-protecting 2-[phenethyl]-5-[(3,4-methylenedioxy)benzyl]pyrrole;

reducing N-protected 2-[phenethyl]-5-[(3,4-methylenedioxy)benzoyl]pyrrole with noble metal catalyst under the mild reaction conditions to N-protected cis 2-[phenethyl]-5-[(3,4-methylenedioxy)-benzoyl]pyrrolidine;

removing N-protecting group from cis 2-[phenethyl]-5-[(3,4-methylenedioxy) benzoyl]-pyrrolidine

reducing cis ±2-[phenethyl]-5-[(3,4-methylenedioxy)benzoyl]pyrrolidine to ±cis erythro 2-[phenethyl]-5-[(3,4-methylenedioxy)-α-hydroxybenzyl]pyrrolidine;

optionally converting cis N-protected 2-[phenethyl]-5-[(3,4-methylenedioxy) benzoyl]pyrrolidine to N-protected ±cis threo 2-[phenethyl]-5-[(3,4-methylenedioxy)-α-hydroxybenzyl]pyrrolidine and subsequently removing N-protecting group;

optionally isomerazing N-protected cis 2-[phenethyl]-5-[(3,4-methylenedioxy)benzoyl]-pyrrolidine to N-protected trans 2-[phenethyl]-5-[(3,4-methylenedioxy)benzoyl]pyrrolidine; and subsequently removing N-protecting group and reducing to ±trans erythro 2-[phenethyl]-5-[(3,4-methylenedioxy)-α-hydroxybenzyl]pyrrolidine.

optionally converting trans N-protected 2-[phenethyl]-5-[(3,4-methylenedioxy) benzoyl]pyrrolidine to N-protected ±trans threo 2-[phenethyl]-

5-[(3,4-methylenedioxy)-α-hydroxybenzyl]pyrrolidine and subsequently removing N-protecting group;

optionally converting all isomers 2-[phenethyl]-5-[(3,4-methylenedioxy)-α-hydroxybenzyl]pyrrolidine to a salt; and

optionally converting salt to a base.

Compounds of the formula (I) may also be prepared by cyclization of the corresponding epoxy and amino substituted straight chain compound. This cyclization is suitably achieved by dissolving the starting compound in aqueous alcohol, such as ethanol-water or methanol-water, treating the resultant solution with excess of strong base, such as preferably sodium or potassium hydroxide, at elevated temperatures, preferably reflux temperatures, for one half to 3 hours, preferably for about an hour. The resulting product may then be isolated using chromatographic procedures.

The starting compound for such cyclization reaction suitably has the formula (I)':

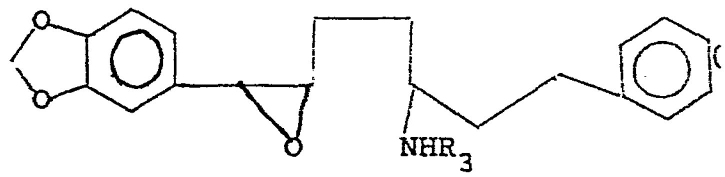 (I)'

wherein $R_3$ is hydrogen, or preferably, a N-protecting group such as trifluoroacetyl.

Thus, in a further aspect, the invention provides a process for preparing a compound of formula (I), or a pharmaceutically acceptable salt thereof, which process comprises either:

(i) the reduction of a compound of formula $(I)_a$:

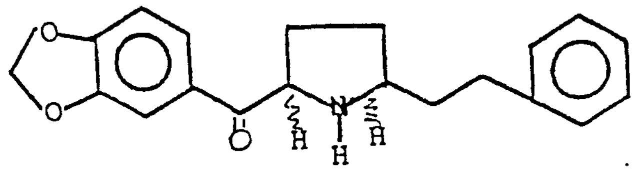

(I)$_a$; or

(ii) the de-protection of a compound of formula (I)$_b$:

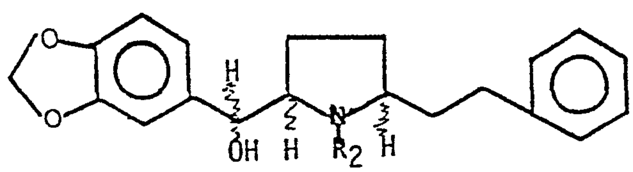

(I)$_b$

wherein $R_2$ is a protecting group; or

(iii) the pyrrolidine forming cyclization of the corresponding epoxy and amino substituted straight chain compound; or

(iv) converting a free base of formula (I) to a salt thereof; or

(v) converting a salt of formula (I) to a free base of formula (I); or

(vi) converting one salt of formula (I) to another salt of formula (I).

The starting compounds for processes (i) and (ii) may be prepared as described herein. The starting compounds for process (iii), such as compounds of formula (I)', may be prepared as described in the Reaction Scheme 2 of European Patent Application No. 82303835.1 (Publication No: 0071399).

## UTILITY AND ADMINISTRATION

### Utility

The compounds of the invention are active antihypertensives. When administered orally or subcutaneously, in very small doses, they relieve hypertension in spontaneously hypertensive rats (SHR) but, at the same time they do not affect rate and force of the heart beat. Accordingly, they are potentially useful as drugs for management of hypertension. They are also active as bronchodilators.

### Administration

Administration of the active compounds and salts described herein can be via any of the accepted modes of administration for cardiovascular system regulating agents. These methods include oral or parenteral routes, such as intravenous, subcutaneous, intradermal, or intramuscular, but preferably oral mode of administration.

Parenteral route of administration is the administration of drugs to a patient by injection under or through one or more layers of the skin or mucous membrane. Parenteral administration would preferably be reserved for crisis situations, wherein the subject is unable to swallow or administer the medication to himself.

The amount of active ingredient administered will, of course, be dependent on the subject being treated, on the severity of the affliction, on the manner of administration and on the judgment of the prescribing physician. However, an effective dosage is in the range of 0.001-50 mg/kg/day, preferably 0.01-1 mg/kg/day. For an average 70 kg human, this would amount to .07-3.500 mg per day, preferably 0.7-70 mg/day.

For oral administration, a pharmaceutically acceptable non-toxic composition is formed by the incorporation of any of the normally employed excipients, such as, for example pharmaceutical grades of mannitol,

lactose, starch, magnesium stearate, sodium saccharin, talcum, cellulose, glucose, sucrose, magnesium, carbonate, and the like. Such compositions take the form of solutions, suspensions, tablets, pills, capsules, powders, sustained release formulations and the like. Such compositions may contain 0.1%-95% of active ingredient, preferably 1%-70%.

For parenteral administration, such as, for example, intravenous injections, the compound is dissolved in a vehicle. Vehicle may be, for example, aqueous vehicle, such as sodium chloride injection, Ringer's injection, dextrose injection and others, water miscible vehicle, such as ethyl alcohol, polyethylene glycol of the liquid series or propylene glycol, or nonaqueous vehicles such a corn oil, peanut oil or sesame oil. Vehicle will be buffered to the proper pH in order to stabilize a solution against chemical degradation and formed in such a way as to control isotonicity of injection. Other substances may also be added as antimicrobial or antioxidant agents.

For use as bronchodilators, administration of the active compounds and salts described herein can be via any of the accepted modes for bronchodilation, i.e., any mode described above can be used and compounds may also be administered in aerosol form.

Pharmaceutical Composition

Depending on the intended mode of administration, the pharmaceutical compositions may be in the form of solid, semi-solid or liquid dosage forms, such as, for example, tablets, pills, capsules, powders, liquids, suspensions, or the like, preferably in unit dosage forms suitable for single administration of precise dosages. The pharmaceutical compositions will include a conventional pharmaceutical carrier or excipient, and a compound of this invention or the pharmaceutically

acceptable salt as an active ingredient thereof. In addition, it may include other medicinal or pharmaceutical agents, carriers, adjuvants, etc.

The composition or formulation to be administered will, in any event, contain a quantity of the active ingredient(s) in an amount effective to alleviate the symptoms of the subject being treated.

For solid pharmaceutical compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like. The active ingredient as defined above may also be formulated as suppositories, using as the carrier for example polyalkylene glycols, such as propylene glycol.

Liquid pharmaceutically administerable compositions can be prepared by dissolving or dispersing, or otherwise preparing an active ingredient (as defined above), and mixing it optionally with a pharmaceutical adjuvant in a carrier, such as, for example, water, saline, aqueous dextrose, glycerol, ethanol, and the like, to thereby form a solution or suspension.

If desired, the pharmaceutical composition to be administered may also contain minor amounts of nontoxic auxiliary substances such as wetting or emulsifying agents, pH buffering agents and the like, such as for example, sodium acetate, sorbitan monolaurate, triethanolamine sodium acetate, sorbitan monolaurate, triethanolamine oleate, etc.

Methods of preparing various pharmaceutical compositions with certain amount of active ingredient are known, or will be apparent, to those skilled in this art. For examples, see <u>Remington's Pharmaceutical Sciences</u>, Mack Publishing Company, Easton, Pennsylvania, 15th Edition (1975).

The Roman numerals in Examples correspond to a Roman numerals in Reaction Schemes and in Preparation Procedures.

EXAMPLE 1

Preparation of 3,4-Methylenedioxybenzoylmorpholide (I)

To a suspension of 11.15 g of 3,4-methylenedioxybenzoic acid (Trans World Chem.) in 250 ml of dichloromethane was added 2.87 ml of thionyl chloride and 1 ml of dimethylformamide. The mixture was stirred at room temperature for approximately 15 min. or until solution occurred. The solvent was removed in vacuo. The residual acid chloride was dissolved in ether, and 5.8 ml of morpholine was added slowly with stirring. The precipitate was removed by filtration. The ether was evaporated in vacuo to give 11.4 g of an oil which was purified by column chromatography in silica gel with ethyl acetate-hexane (2:3) as the eluting solvent. 3,4-methylenedioxybenzoylmorpholide (I) was obtained. (Step 3)

EXAMPLE 2

Preparation of Phenylacetylmorpholide (IIa)

4 ml of thionyl chloride and 0.5 ml of dry dimethylformamide were added to a solution of 12.5 g of phenylacetic acid (Sigma) in 200 ml of dry dichloromethane. The solution was stirred for 15 minutes and evaporated to dryness in vacuo. The residual acid chloride was dissolved in 100 ml of dry dichloromethane. A solution of 5.35 ml of morpholine in 100 ml of dichloromethane was added dropwise with stirring. When the addition was ended, the mixture was evaporated to dryness in vacuo and the residue was percolated through a short silica gel column using ethyl acetate-hexane (3:7)

as the percolating solvent. 13.7 g. of phenylacetylmorpholide (IIa) was obtained, m.p. 64-66°C. (Step 1)

EXAMPLE 3

Preparation of 2-Phenylacetylpyrrole (III)

Vilsmeier-Haack reaction was carried out according to the method of J. White and G. McGillivray, J. Org. Chem., 42, 4248 (1979).

A mixture of 30 g (0.146 mole) of phenylacetylmorpholide and 27 ml (0.295 mole) of phosphorous oxychloride was stirred magnetically at room temperature in a nitrogen atmosphere for 6 h. A solution of 10 ml (0.149 mole) of pyrrole in 700 ml of anhydrous 1,2-dichloroethane was added at a rate such that the temperature did not exceed 30°. The reaction mixture was stirred at room temperature for 18 h. The mixture was cautiously mixed with a solution of 700 ml of 10% sodium carbonate in water and the mixture was heated at reflux temperature with stirring for 1.5 h. The cooled mixture was filtered through celite. The organic phase was separated and combined with dichloromethane extracts (3x500 ml) of the aqueous phase, the organic phases were dried over sodium sulfate and evaporated *in vacuo*. The residue was subjected to column chromatography over silica gel (1 kg) 9.6 g (35%) of 2-phenylacetylpyrrole (III) was eluted with dichloromethane and crystallized from acetone-hexane, m.p. 92-94°. (Step 1)

EXAMPLE 4

Preparation of 2-[Phenethyl]Pyrrole (IV)

A solution of 6.00 g (0.032 mole) of the 2-phenylacetylpyrrole (III) in 200 ml of anhydrous tetrahydrofuran was added to a suspension of 600 g (0.153 mole) of lithium aluminum hydride in dry tetrahydrofuran. The mixture was stirred at reflux

temperature for 48 h. The mixture was cooled to 0°, ethyl acetate was cautiously added to destroy the excess hydride and then saturated aqueous sodium sulfate was added. The organic phase was decanted, dried over sodium sulfate and evaporated in vacuo. The residue was subjected to column chromatography on neutral alumina (Fluka, Act II). 4.800 g (88%) of 2-[phenethyl]pyrrole (IV) was eluted with hexane-ethyl acetate and crystallized, m.p. 40-42° (hexane). (Step 2).

EXAMPLE 5

Preparation of 2-[Phenethyl]-5-[(3,4-Methylenedioxy)Benzoyl]Pyrrole (V)

A mixture of 12.3 g (0.046 mole) of [(3,4-methylenedioxyObenzoyl]morpholide (I) and 12 ml (0.13 mole) of phosphorous oxychloride was stirred at room temperature for 3 h in a nitrogen atmosphere. A solution of 10.0 g (0.058 mole) of 2-[phenethyl]pyrrole (IV) in 200 ml of dry 1,2-dichloroethane was added and the mixture was stirred at room temperature for 18 h. The reaction mixture was stirred at room temperature for 18 h. The mixture was cautiously mixed with a solution of 700 ml of 10% sodium carbonate in water and the mixture was heated at reflux temperature with stirring for 1.5 h. The cooled mixture was filtered through celite. The organic phase was separated and combined with dichloromethane extracts (3x500 ml) of the aqueous phase, the organic phases were dried over sodium sulfate and evaporated in vacuo. The residue was submitted to column chromatography on neutral alumina (Fluka, Act II). The crude product was purified by column chromatography on silica gel (1 kg). The desired material was eluted with dichloromethane to give a 5.58 g (38%) of 2-[phenethyl]-5-[(3,4-methylenedioxy)-benzoyl]pyrrole (V) which was crystallized from

dichloromethane-acetone, m.p. 143-145° (dichloromethane-acetone). (Step 3)

EXAMPLE 6

Preparation of 1-t-Butoxycarbonyl-2-[Phenethyl]-5-[(3,4-Methylenedioxy)Benzoyl]Pyrrole (VI)

4.10 g (0.012 mole) of 2-[phenethyl]-5-[(3,4-methylenedioxy)benzoyl]pyrrole (V) was added to a 1.40 g of suspension (0.05 mole; 60% dispersion in mineral oil) of sodium hydride in 100 ml of dry dimethylformamide. The mixture was heated at 45-60° for 2 h. 4.51 g (0.02 mole) of di-t-butyl dicarbonate was added rapidly and the solution was stirred at 60-70° for 2 h. The reaction mixture was cooled, poured onto ice-water and the product was extracted into ethyl acetate. The extract was washed with water, dried over sodium sulfate and evaporated in vacuo. Then it was purified by column chromatography on alumina (300 g, Fluka, Neutral Act. II). The crude product was crystallized from acetone-hexane to give the 5.0 g (94%) of 1-t-butoxycarbonyl-2[phenethyl]-5-[(3,4-methylenedioxy)benzoyl]pyrrole (VI), m.p. 145-147° (acetone-hexane). (Step 4)

EXAMPLE 7

Preparation of 1-t-Butoxycarbonyl Cis 2-[Phenethyl]-5-[(3,4-Methylenedioxy)Benzoyl)Pyrrolidine (VII)

A solution of 4.50 g, (0.01 mole) 1-t-butoxycarbonyl-2[phenethyl]-5-[(3,4-methylenedioxy)benzoyl]pyrrole (VI) in 300 ml of ethanol-ethyl acetate (2:1) containing 1.8 g of suspended platinic oxide was hydrogenated at room temperature and atmospheric pressure for 18 h. The reaction mixture was filtered, the filtrate was evaporated in vacuo and the residue was subjected to column chromatography on neutral alumina (Fluka, Act II). The product was eluted with hexane-ethyl

acetate (95:5). The crude 1-t-butoxycarbonyl cis 2-[phenethyl]-5-[(3,4-methylenedioxy)benzoyl]pyrrolidine (VII) was obtained in quantitative yield as an oil. Oil. (Step 5)

Oil

| | | |
|---|---|---|
| U.V. | (MeOH) | 215, 221 sh, 233, 276, 288 sh, 311, 299 sh nm (ε 8910; 9330; 13,200; 6030); 4270; 6760; 5370). |
| I.R. | ($CHCl_3$) 1756, EM-390 | 1.37 (s, 9H, t-butyl<br>1.48-1.86 (m, 5H)<br>2.28 (m, 1H)<br>2.71 (m, 2H)<br>4.00 (m, 1H<br>5.20 (m, 1H)<br>6.01 (s, 2H, )$CH_2$O)<br>6.83 (d, 1H, J = 7.5 Hz, aromatic hydrogen)<br>7.25 (s, 5H, aromatic hydrogens)<br>7.75 (m, 2H, aromatic hydrogens. |

## EXAMPLE 8

### Preparation of Cis 2-[Phenethyl]-5-[(3,4-Methylene-Dioxy)Benzoyl]Pyrrolidine Trifluoroacetate (VIII)

50 ml of trifluoroacetic acid was added to a solution of 3.60 g (0.0085 mole) of 1-t-butoxycarbonyl-cis 2-[phenethyl]-5-[(3,4-methylenedioxy)benzoyl]-pyrrolidine (VII) in 200 ml of dry dichloromethane. The reaction solution was stirred at room temperature for 0.5 h. The solvent was removed *in vacuo* and the residue was crystallized from dichloromethane-ether to give 2.90 g (73%) of cis 2-[phenethyl]-5-[(3,4-methylene-dioxy)benzoyl]pyrrolidine trifluoroacetate (VIII), m.p. 166-168° (dichloromethane-ether). (Step 6)

EXAMPLE 9

Preparation of ±Cis Erythro 2-[2-Phenethyl]-5-[(3,4-Methylenedioxy)-α-hydroxybenzyl]-Pyrrolidine (IX)

1.35 g (0.035 mole) of sodium borohydride was added to a stirred solution of 2.70 g (0.0057 mole) of cis 2-[phenethyl]-5-[(3,4-methylenedioxy)benxoyl]-pyrrolidine in 270 ml ethanol, at 0° temperature. After 1 hour at 0°, the mixture was poured into 100 ml of 10% ammonium chloride solution. The mixture was evaporated in vacuo to remove the ethanol, the residue was cooled to 0°, and 50 ml of a saturated sodium carbonate solution was added. The product was extracted into ethyl acetate, the extract was washed with water, dried over sodium sulfate and evaporated in vacuo. The residue was crystallized from ethyl acetate-hexane to give 1.72 g (93%) of the desired ±cis erythro 2-[phenethyl]-5-[(3,4-methylenedioxy)-α-hydroxybenzyl]pyrrolidine (IX), m.p. 122-123° (dichloromethane-hexane). (Step 7)

EXAMPLE 10

Preparation of ±Cis Erythro and ±Cis Threo 1-t-Butoxycarbonyl-2-[Phenethyl]-5-[(3,4-Methylene-Dioxy)α-Hydroxybenzyl]Pyrrolidine (X) and (XI)

A solution of 3.20 g (4.5 mmole) of the cis 1-t-butoxycarbonyl-2[phenethyl]-5-[(3,4-methylenedioxy)-benzoyl]pyrrolidine (VII), prepared in Example 7 and 6.4 g (16.9 mmole) of sodium borohydride in 300 ml of ethanol is heated at a reflux temperature for 45 min. The solvent is removed in vacuo and the residue is partitioned between water and ethyl acetate. The organic phase is evaporated in vacuo and the residue is percolated through a short column of silica gel using ethyl acetate-hexane (1:3) as the percolating solvent.

The resulting mixture is separated by TLC with ethyl acetate/hexane (1:3) into two isomers:

±cis erythro 1-t-butoxycarbonyl-2-[phenethyl]-5-[(3,4-methylenedioxy)-α-hydroxybenzyl]pyrrolidine and

±cis threo 1-t-butoxycarbonyl-2-[phenethyl]-5-[(3,4-methylenedioxy)-α-hydroxybenzyl]pyrrolidine. (Step 8)

The same procedure is used for the preparation of trans erythro (XVIII) and trans threo (XVI) compounds (shown in Reaction Scheme 4). (Step 14)

The resulting compounds are:

±trans erythro 1-t-butoxycarbonyl-2-[phenethyl]-5-[(3,4-methylenedioxy)-α-hydroxybenzyl]pyrrolidine and

±trans threo 1-t-butoxycarbonyl-2-[phenethyl]-5-[(3,4-methylenedioxy)-α-hydroxybenzyl]pyrrolidine.

## EXAMPLE 11

### Removal of N-Protecting Group from ±Cis Erythro or ±Cis Threo 1-t-Butoxycarbonyl-2-[Phenethyl]-5-[(3,4-Methylenedioxy)-α-Hydroxybenzyl]Pyrrolidine (X)(XI)

50 ml of trifluoroacetic acid is added to a solution of ±cis erythro 1-t-butoxycarbonyl-2-[phenethyl]-5-[(3,4-methylenedioxy)-α-hydroxybenzyl]pyrrolidine (X) or to a solution of 3.60 g (0.0085 mole) ±cis threo 1-t-butoxycarbonyl-[phenethyl]-5-[(3,4-methylenedioxy)-α-hydroxybenzyl]pyrrolidine (XI) in 200 ml of dichloromethane. The reaction mixture is stirred at room temperature for 0.5-1 hour. The solvent is removed *in vacuo* and the residue is crystallized from dichloromethane-ether to give trifluoroacetic acid salt of compounds (X) and (XI), resulting in:

±cis erythro 2-[phenethyl]-5-[(3,4-methylenedioxy)-α-hydroxybenzyl]pyrrolidine trifluoroacetate (IX);and

±cis threo 2-[phenethyl]-5-[(3,4-methylenedioxy)-α-hydroxybenzyl]pyrrolidine trifluoroacetate (XII). (Step 9)

The same procedure is used for the removal of N-protecting group from compounds (XIII), (XVI), and (XVIII), shown in Reaction Schemes 3 and 4.

EXAMPLE 12

Preparation of Trans 1-t-Butoxycarbonyl-2-[Phenethyl]-5-[(3,4-Methylenedioxy)Benzoyl] Pyrrolidine (XIII)

A solution of 50 g of cis pyrrolidine (VII) prepared in Example 7 in a solvent mixture consisting of 2.25 ml of 2-propanol and 100 ml of 10% aqueous sodium hydroxide is heated at reflux temperature for 26 hours. The solution is evaporated to dryness, water is added to the residue and the mixture is extracted with ethyl acetate. The solvent is evaporated and the residue is separated by column chromatography (silica gel/1 Kg/one meter column). The column is developed with ethyl acetate/hexane (1:4) to give, as the first fraction, the trans 1-t-butoxycarbonyl-2-[phenethyl]-5-[(3,4-methylenedioxy)benzoyl]pyrrolidine (XIII) and as a second fraction nonconverted cis isomer (VII).

EXAMPLE 13

Conversion of Free Base to Salt

Preparation of 2-[Phenethyl]-5-[(3,4-Methylenedioxy) α-Hydroxybenzyl]Pyrrolidine Hydrochloride

Excess 3% hydrogen chloride in methanol is added to a solution of 1.0 g of 2-[phenethyl]-5-[(3,4-methylenedioxy)-α-hydroxybenzyl]pyrrolidine in 20 ml of methanol. Diethyl ether is added until precipitation is complete. 2-[phenethyl]-5-[(3,4-methylenedioxy)-α-hydroxybenzyl]pyrrolidine hydrochloride is filtered, washed with ether, air dried and recrystallized.

## EXAMPLE 14

### Conversion of Salt to Free Base

### Preparation of 2-[Phenethyl]-5-[(3,4-Methylenedioxy)-α-Hydroxybenzyl]Pyrrolidine

1.0 g of 2-[phenethyl]-5-[(3,4-methylenedioxy)-α-hydroxybenzyl]pyrrolidine hydrochloride or trifluoroacetate (XII') and (XVII') is dissolved in 50 ml of water. A solution of sodium bicarbonate is added, and the pH adjusted to about pH 5. The resulting free base is extracted with ethyl acetate, the organic layer is then separated, washed twice with water, dried over magnesium sulfate and evaporated to yield 2-[phenethyl]-5-[(3,4-methylenedioxy)-α-hydroxybenzyl)]pyrrolidine as the free base.

## EXAMPLE 15

### Direct interchange of acid addition salts

1 g of 2-[phenethyl]-5-[(3,4-methylenedioxy)-α-hydroxybenzyl]pyrrolidine acetate, prepared according to Example 15, is dissolved in a solution of 1 ml 50% aqueous sulfuric acid in 10 ml ethanol and the resulting precipitate is harvested. The product is suspended in ethanol and filtered, air dried, and recrystallized from methanol/acetone to yield 2-[phenethyl]-5-[(3,4-methylenedioxy)-α-hydroxybenzyl]pyrrolidine bisulfate.

In Examples 16-23, the active ingredient is ±cis erythro 2-[phenethyl]-5-[(3,4-methylenedioxy)-α-hydroxybenzyl]pyrrolidine hydrochloride.

EXAMPLE 16

| Ingredients | Quantity per tablet, mgs. |
| --- | --- |
| Active ingredient | 25 |
| cornstarch | 20 |
| lactose, spray-dried | 153 |
| magnesium stearate | 2 |

The above ingredients are thoroughly mixed and pressed into single scored tablets.

EXAMPLE 17

| Ingredients | Quantity per tablet, mgs. |
| --- | --- |
| Active ingredient | 100 |
| lactose, spray-dried | 148 |
| magnesium stearate | 2 |

The above ingredients are mixed and introduced into a hard-shell gelatin capsule.

EXAMPLE 18

| Ingredients | Quantity per tablet, mgs. |
| --- | --- |
| Active ingredient | 200 |
| cornstarch | 50 |
| lactose | 145 |
| magnesium stearate | 5 |

The above ingredients are mixed intimately and pressed into single scored tablets.

## EXAMPLE 19

| Ingredients | Quantity per tablet, mgs. |
|---|---|
| Active ingredient | 108 |
| lactose | 15 |
| cornstarch | 25 |
| magnesium stearate | 2 |

The above ingredients are mixed and introduced into a hard-shell gelatin capsule.

## EXAMPLE 20

| Ingredients | Quantity per tablet, mgs. |
|---|---|
| Active ingredient | 150 |
| lactose | 92 |

The above ingredients are mixed and introduced into a hard-shell gelatin capsule.

## EXAMPLE 21

An injectable preparation buffered to a pH of 7 is prepared having the following composition:

| Ingredients | |
|---|---|
| Active ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

EXAMPLE 22

An oral suspension is prepared having the following composition:

| Ingredients | |
|---|---|
| Active ingredient | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water | q.s. to 100 ml |

EXAMPLE 23

| | |
|---|---|
| Active ingredient | 3.0% |
| Span[R] 85 (sorbitan trioleate) | 1.0% |
| Freon 11 (trichloromonofluoromethane) | 30.0% |
| Freon 114 (dichlorotetrafluoroethane) | 41.0% |
| Freon 12 (dichlorodifluoromethane) | 25.0% |

For the sake of completeness, the following Examples A to H are included as they set out essentially the same procedures as in Examples 1 to 9 hereinbefore, but using the presentation and terminology of PC T/US83-00098. The product cis-erythro-2-[3,4-methylenedioxy-(1-hydroxy benzyl)]-5-phenylethyl pyrrolidine of following Example H is, of course, the same compound as ±cis erythro 2-[phenethyl]-5-[(3,4-methylenedioxy)-α-hydroxybenzyl]-pyrrolidine, the product of Example 9 hereinbefore.

## EXAMPLE A

### Preparation of Model Compound 4-Benzyloxyphenyl-acetylmorpholide

Thionyl chloride (4 ml) and dry dimethylformamide (0.5 ml) were added to a solution of 4-benzyloxyphenyl-acetic acid (12.5 g) in dry dichloromethane (200 ml). The solution was stirred for 15 min., evaporated to dryness *in vacuo* and the residual acid chloride was dissolved in dry dichloromethane (100 ml). A solution of morpholine (5.35 ml) in dry dichloromethane (100 ml) was then added dropwise with stirring. When the addition was ended the mixture was evaporated to dryness *in vacuo* and the residue was percolated through a short silica gel column using ethyl acetate-hexane (3:7) as the percolating solvent. A crystalline solid 4-benzyloxy-phenylacetylmorpholide (13.7 g), was obtained, m.p. 119-120°C.

Similarly, the following compound may be prepared: 3,4-methylenedioxyphenylacetyl morpholide.

## EXAMPLE B

### Preparation of Model Compound 2-(4-Benzyloxy-phenylacetyl)pyrrole

A mixture of the morpholide (1.0 g) and phosphorous oxychloride (1.53 g) was stirred at room temperature for 6 hours. To this material was added a solution of pyrrole (3 ml) in 1,2-dichloroethane (15 ml) and the mixture was stirred for 15 hours at room temperature. A 20% aqueous sodium bicarbonate solution (10 ml) was added in a dropwise manner and when the addition was completed the mixture was heated at reflux temperature for 1 hour. The organic phase was separated, dried and evaporated *in vacuo*. The residue was subjected to chromatography on silica gel (80 g) the product (0.60 g) being eluted with dichloromethane.

2-(4-Benzyloxyphenylacetyl)pyrrole was crystallized from dichloromethane-ethanol, m.p. 136-137°C.

Similarly, the following compound may be prepared: 2-Phenylacetylpyrrole, m.p. 92-94°C.

EXAMPLE C

Model Compound 2-(4-Benzyloxyphenethyl)pyrrole

To a solution of the 2-(4-Benzyloxyphenylacetyl)-pyrrole (1.0 g) in dry tetrahydrofuran (80 ml), cooled to 0°C, was added lithium aluminium hydride (0.56 g). After stirring at 0°C for 30 min. the mixture was heated at reflux temperature for 15 hours. It was then cooled to 0°C and neutral alumina hydrated with 3 wt.% water was added to decompose the excess hydride reagent. Ethyl acetate was then added and the mixture was filtered through Celite. The solvent was evaporated and the residual material was percolated through a short column of alumina using ethyl acetate-hexane (5:95) as the percolating solvent. This procedure gave the desired product 2-(4-benzyloxyphenethyl)pyrrole (0.55 g, 60%) after crystallization from dichloromethane-hexane, m.p. 102-103°C.

Similarly, the following compound can be prepared: 2-(2-Phenethyl)pyrrole, m.p. 40-42°C.

EXAMPLE D

Model Compound 2-(3,4-Dibenzyloxybenzoyl)-5-(4-Benzyloxy-phenethyl)pyrrole

A mixture of the 3,4-dibenzyloxybenzoylmorpholide (1.0 g) and phosphorous oxychloride (5 ml) was stirred at room temperature for 6 hours. The excess phosphorous oxychloride was removed with a stream of nitrogen and a solution of 2-(4-benzyloxyphenethylpyrrole (Preparation 26) (0.50 g) in 1,2-dichloroethane (10 ml) was added to the residual Vilsmeier-Haack reagent. The

mixture was stirred for 15 hours at room temperature and then an excess of 20% aqueous potassium carbonate was added. The mixture was heated at reflux temperature for 3 hours, the organic phase was separated, dried and evaporated *in vacuo*. The solid residue was washed with ether to yield after crystallization from chloroform-ether a white solid 2-(3,4-dibenzyloxybenzoyl)-5-(4-benzyloxyphenethyl)pyrrole (0.66 g), m.p. 173-175°C.

Similarly, the following compound may be prepared:

2-(3,4-methylenedioxybenzoyl)-5-(2-phenethyl)pyrrole, m.p. 143-145°C.

## EXAMPLE E

## Preparation of Model Compound 1-t-Butoxycarbonyl-2-(3,4-Dibenzyloxybenzoyl)-5-(4-Benzyloxyphenylethyl)pyrrole

2-(3,4-Dibenzyloxybenzoyl)-5-(4-benzyloxyphenylethyl)-pyrrole 1 (10.0 g, 16.8 mmole) dissolved in anhydrous dimethylformamide (250 ml) was added dropwise to a stirred suspension of 60% sodium hydride (0.808g, 20.2 mole) in dimethylformamide at 40°. When the formation of the anion was complete a solution of di-t-butyl dicarbonate (7.35 g, 33.7 mmole) in dry dimethylformamide (165 ml) was added and the solution was then stirred at 65° for 4 hours. The solution was diluted with ethyl acetate and washed with water. The organic phase was dried over sodium sulfate and evaporated *in vacuo*. The crude product was subjected to column chormatography on silica gel (400 g) the product being eluted with ethyl acetate-hexane (1:4). The product which was obtained (9.35 g, 80%) was crystallized from dichloromethane-hexane to give a solid with mp 128-129°.

Similarly may be prepared:

1-t-Botoxycarbonyl-2-(3,4-methylenedioxybenzoyl)-5-phenethylpyrrole, m.p. 145-147°C.

EXAMPLE F

Preparation of cis 1-t-Butoxycarbonyl-2-(3,4-methylene dioxybenzoyl)-5-phenethyl pyrrolidine

A solution of the corresponding pyrrole (4.50 g, 0.01 mole) in 2:1 ethanol-ethyl acetate (300 ml) containing suspended platinic oxide (1.8 g) was hydrogenated at room temperature and atmospheric pressure for 18 hours. The reaction mixture was filtered, the filtrate evaporated in vacuo and the residue was subjected to column chromatography on neutral alumina to give the crude product in quantitative yield as an oil.

EXAMPLE G

Model Compound Cis-2-(3,4-dibenzyloxybenzoyl)-5-(4-benzyloxyphenethyl)-pyrrolidine trifluoroacetic acid salt.

Trifluoracetic acid (50 ml) was added to a solution of 1-t-butoxycarbonyl-cis-2-(3,4-methylene dioxybenzoyl)-5-phenethylpyrrolidine (3.60 g, 0.0085 mole) in dry dichloromethane (200 ml). The reaction solution was then stirred at room temperature for 0.5 h, the solvent was removed in vacuo and the residue was crystallized from dichloromethane-ether to give 8b (2.90 g, 73%) as the trifluoroacetic acid salt, m.p. 166 - 168°.

Similarly may be prepared:

Cis-2-(2-phenylethyl)-5-(3,4-methylenedioxybenzoyl)-pyrrolidinium trifluoroacetate acid salt.

EXAMPLE H

Preparation of Cis-Erythro-2-[3,4-methylenedioxy-(1-Hydroxybenzyl)]-5-Phenylethyl Pyrrolidine

Sodium borohydride (1.35 g, 0.035 mole) was added to a stirred solution of cis-2-(3,4-methylenedioxybenzyoyl)-5-phenylethyl pyrrolidine trifluoroacetate (2.70 g, 0.0057 mole) in ethanol (270 ml) at 0°. After 1 h at 0°, the mixture was poured into 10% ammonium chloride solution (100 ml), the mixture was evaporated *in vacuo* to remove the ethanol, the residue was cooled to 0° and a saturated sodium carbonate solution (50 ml) was added. The product was extracted into ethyl acetate, the extract was washed with water, dried over sodium sulfate and evaporated *in vacuo*. The residue was crystallized from ethyl acetate-hexane to give the desired cis erythro cis-erythro-2-[3,4-methylenedioxy-(1-Hydroxybenzyl)]-5-phenylethyl pyrrolidine (1.72 g, 93%), m.p. 122-123°.

EXAMPLE 24

Antihypertensive Activity of ±Cis Erythro 2-[Phenethyl]]-5-[(3,4-Methylenedioxy) α-Hydroxybenzyl]Pyrrolidine

This example illustrates superior antihypertensive activity of compounds of this invention. In this Example, I, II, III, or IV mean compounds as shown in Tables 1 and 2.

24 previously trained adult male spontaneously hypertensive rats were distributed into 6 groups (5 animals per group) with approximately equal mean systolic blood pressures. The 6 groups were then studied concurrently in a 2-day compound screening procedure.

Test compounds were randomly assigned to each group. 5 groups received potential antihypertensive agents and 1 control group received vehicle only (water and Tween).

At approximately 17 hours prior to the first day of dosing food was removed from the rat cages. On the morning of Day 1, a group of 4 rats was orally dosed (by gavage) with 12.5 mg/kg or 25 mg/kg of ±cis erythro 2-[phenethyl]-5-[(3,4-methylenedioxy)]-α-hydroxybenzyl)-pyrrolidine (I) or other tested compound dissolved/suspended in water (using 2-3 drops Tween 80) with a homogenizer at concentrations such that 0.1 ml of solution was administered per 10 g of body weight. At 4 1/2 hours post dose, food was put back in the cages and the rats were allowed to eat for 2-1/2 hours, after which food was again removed. On the morning of Day 2, rats were orally dosed as described above. Immediately after dosing, the rats were put in restrainers and placed in a heated chamber (30 ± 1.0°C) for four hours. Normal feeding resumed at the end of the study on Day 2.

Systolic blood pressure (i.e., pressure at the appearance of the first pulse) were recorded using photoelectric transducers. The coccygeal arteries of 3 rats (in a horizontal group) were simultaneously occluded by pump-inflated tail cuffs that were automatically inflated to 300 mmHg and then deflated. A pressure curve and tail pulses were simultaneously monitored on an MFE recorder. Four consecutive (at 30 second intervals) traces were recorded for each rat in a given horizontal group at one, two, three and four hours post compound administration. Subsequent horizontal groups were automatically tested in the same manner.

The mean systolic blood pressure (SBP) of each rat at each observation time was calculated. The SBP of the animals in each dose group were compared to the SBP of the animals in the control group (vehicle only) at each observation time using a one-way analysis of variance test. A compound exhibiting p0.05 at any observation time was considered to exhibit significant

antihypertensive activity. Compounds significantly decreasing blood pressure 20 mmHg or more from control values at all four observation times were considered worthy of further examination. In these instances heart rates were calculated and tested for significant change from control heart rate values using the two-tailed test. Pressures were read at hours 1, 2, 3 and 4 after dosing on both days 1 and 2.

A. Table 1 summarizes the results obtained by testing the compounds, namely, ±cis erythro ±-2-[phenethyl]-5-[(3,4-methylenedioxy)-α-hydroxybenzyl]pyrrolidine (I); ±cis erythro 2-[phenethyl]-5-[(3,4-dihydroxy)-α-hydroxybenzyl]-pyrrolidine (II) and ± trans erythro-2-[phenethyl]-5-[(3,4-dihydroxyphenyl)-α-hydroxymethyl]pyrrolidine (III).

Table 1

| Compound | | ±cis erythro 2-[phenethyl]-5-[(3,4-methylenedioxy)-α-hydroxybenzyl]pyrrolidine | | | | | | ±cis erythro 2-[phenethyl]-5-[(3,4-dihydroxy)-α-hydroxybenzyl]pyrrolidine | | | | | | ±trans erythro 2-[phenethyl]-5-[(3,4-dihydroxy)-α-hydroxybenzyl]pyrrolidine | | | | | |
|---|---|---|---|---|---|---|---|---|---|---|---|---|---|---|---|---|---|---|---|
| | | Compound I | | | | | | Compound II | | | | | | Compound III | | | | | |
| Dose | | per os 25 mg/kg | | | | | | per os 25 mg/kg | | | | | | per os 25 mg/kg | | | | | |
| Test | | Systolic Blood Pressure | | | Heart Rates | | | Systolic Blood Pressure | | | Heart Rates | | | Systolic Blood Pressure | | | Heart Rates | | |
| | | % | p | mm Hg | % | p | BPM | % | p | mm Hg | % | p | BPM | % | p | mm Hg | % | p | BP. |
| Hours | 1 | -35 | 0.001 | -80 | 2 | NS | 5 | -27 | 0.05 | -54 | 47 | 0.05 | 148 | -31 | 0.01 | -62 | 24 | 0.02 | 7 |
| Past | 2 | -33 | 0.03 | -69 | -4 | NS | -12 | -40 | 0.05 | -87 | 41 | 0.05 | 125 | -27 | 0.01 | -54 | 32 | 0.01 | 8 |
| Dosing | 3 | -35 | 0.03 | -72 | -6 | NS | -17 | -43 | 0.05 | -89 | 29 | 0.05 | 90 | -18 | 0.02 | -34 | 10 | NS | 2 |
| | 4 | -35 | 0.01 | -74 | -18 | NS | -55 | -39 | 0.05 | -79 | 32 | 0.05 | 96 | -31 | 0.001 | -69 | -1 | NS | - |

BPM means beats per minute;

ΔmmHg means difference in blood pressure expressed in mmHg;

%Δ means difference in blood pressure expressed in %;

p = significance.

Table 1 illustrates the superiority of compound I ±cis erythro 2-[phenethyl]-5-[(3,4-methylenedioxy)-α-hydroxybenzyl]pyrrolidine over compounds II cis erythro ±2-[phenethyl]-5-[3,4-dihydroxy)-α-hydroxybenzyl]pyrrolidine and III trans erythro 2-[phenethyl]-5-[3,4-dihydroxy)-α-hydroxybenzyl)-pyrolidine.

Compound I significantly decreases systolic blood pressure without, at the same time, increasing the heart rate. Increasing the heart rate is an undesirable condition very often associated with decreasing of blood pressure.

Compound II also significantly decreases systolic blood pressure. However, the decrease in systolic blood pressure caused by compound II is accompanied by very high increase in the heart rate. Increased heart beat persists during all four hours.

Compound III also significantly decreases blood pressure although not as much and not as steadily as compound I. However, the decrease in systolic blood pressure caused by compound III is accompanied by significant increase in the heart beat during the first two hours after dosing.

B. Table 2 summarizes the results obtained by testing two compounds, namely, ±cis erythro 2-[phenethyl]-5-[(3,4-methylenedioxy)-α-hydroxybenzyl]pyrrolidine I and ±cis erythro 2-[(3,4-di-methoxy)phenethyl]-5-[(3,4-methylenedioxy)-α-hydroxybenzyl]-pyrrolidine (IV).

Table 2

| Compound | ±cis erythro 2-[phenethyl]-5-[(3,4-methylenedioxy)-α-hydroxybenzyl]pyrrolidine | | | | | | ±cis erythro 2-[(3,4-dimethoxy)-phenethyl]-5-[(3,4-methylenedioxy)-α-hydroxybenzyl]pyrrolidine | | | | | |
|---|---|---|---|---|---|---|---|---|---|---|---|---|
| | Compound I | | | | | | Compound IV | | | | | |
| Dose | per os | | | 12.5 mg/kg | | | per os | | | 12.5 mg/kg | | |
| Test | Systolic Blood Pressure | | | Heart Rates | | | Systolic Blood Pressure | | | Heart Rates | | |
| | % | p | mm Hg | % | p | BPM | % | p | mm Hg | % | p | BPM |
| Hours Past Dosing 1 | -25 | NS | -48 | 18 | NS | 52 | -9 | NS | -17 | 15 | NS | +45 |
| 2 | -26 | 0.05 | -51 | 5 | NS | 13 | -19 | 0.05 | -42 | 12 | NS | +34 |
| 3 | -29 | 0.05 | -61 | -9 | NS | -28 | -16 | NS | -33 | -4 | NS | +13 |
| 4 | -70 | 0.05 | -91 | -13 | 0.05 | -39 | -25 | 0.05 | -57 | -12 | 0.05 | -34 |
| 8 | -18 | 0.05 | -39 | +2 | NS | 7 | -17 | NS | -35 | -1 | NS | -2 |
| 12 | -2 | NS | -4 | +5 | NS | 13 | -11 | NS | -18 | -8 | NS | -21 |

%Δ, p, ΔmmHg and BPM are as in Table 1.

Compound I cis erythro 2-[phenethyl]-5-[(3,4-methylenedioxy)-α-hydroxybenzyl]pyrrolidine and compound IV, ±cis erythro 2-[3,4-dimethoxy)phenethyl]-5-[(3,4-methylenedioxy)-α-hydroxybenzyl]pyrrolidine were tested using the same antihypertensive test as described above. Only half of the dose used in Table 1, i.e., 12,5 mg/kg dosage was used.

Compound I shows, even at the low dosage of 12,5 mg/kg, a very strong blood pressure lowering effect which lasts more than eight hours following the single dosage. From the second hour until the eighth hour the decrease in systolic blood pressure is significant. Although some increase in the heart rate was observed during the first two hours, this increase was not significant and it quickly normalized. During the third and fourth hour, the heartbeat actually decreased and from 4-12 hours the heartbeat was close to normal.

Compound IV, on the other hand had much less effect on lowering of the systolic blood pressure. Heart rates, while higher in absolute numbers than those of compound I, were also not significantly increased.

Conclusion

Among the four compounds tested compound I has the surprising and unexpectedly high effect on lowering of systolic blood pressure which is not accompanied simultaneously with the increase in the heart rate. The two other compounds II and III also show effect on lowering of systolic blood pressure. Both of them, however, at the same time, increase heart rates. Such increase is undesirable. It forces the heart to pulsate faster forcing the higher volumes of the blood into the peripheral circulation. More rapidly pulsating heart is susceptible to exhaustion and cardiac failure. Also, increased blood volume pumped more rapidly to the periphery can cause increased blood pressure by way of the feedback.

Compound IV has only mild systolic blood pressure decreasing effect. At the same time, however, it slightly increases heart rates.

Thus among the four compounds, compound I is the only compound which lowers the systolic blood pressure significantly and, at the same time, does not increase the heart rate. These two features make compound I desirable antihypertensive.

EXAMPLE 25

Postural Hypotensive Effects of ± Cis Erythro 2-[Phenethyl]-5-[(3,4-Methylenedioxy)-α-Hydroxybenzyl]Pyrrolidine

This example illustrates postural hypotensive effect of compounds of this invention.

Male normotensive Sprague-Dawley rats (Charles River) weighing 250-300 g were used. Surgery was conducted when the rats were under ether anesthesia. The left femoral artery and vein were cannulated for the measurement of blood pressure and for drug administration, respectively. The rats were secured on their backs to a special tilt table and allowed to recover from anesthesia. Animals were tilted from the horizontal to the head-up vertical position (90°) for 2 minutes twice at 15 minute intervals during the control period and at 15, 30, 45, 60 min. after drug administration.

Compounds were prepared in water and were administered orally by gavage. Injection volume was 1 ml/100g. Each rat received only a single dose of a compound.

The hypotensive response is the maximal mean blood pressure lowering induced by the drug with the animal in the supine position. The postural hypotensive effect is the maximal drop in MBP in response to tilting (i.e., the

net drop in BP in reference to that prior to tilt). Responses are expressed as absolute changes in mmHg.

The results are shown in Figure 1.

±Cis erythro 2-[[phenethyl]-5-[(3,4-methylenedioxy)-α-hydroxybenzyl]pyrrolidine induced a dose-dependent hypotensive response (1-100 mg/kg, p.o.). At 10 mg/kg (p.o.), the compound lowered blood pressure 45 mmHg and at 100 mg/kg (p.o.), magnitude of 20-30 mmHg within the dose range tested.

Thus, the compound I is, in a very small dose, very effective in lowering blood pressure in normotensive rats. This finding supports the results shown in Example 24 and the conclusion that Compound I is a desirable antihypertensive.

Toxicity

The same compound of the invention (as the hydrochloride salt) was administered to five groups of five male mice at different doses per group, intravenously as an aqueous solution, and the mice observed for acute and delayed lethality. In this test the I.V. LD50 of the compound was 27 mg/kg.

CLAIMS

1. A compound of formula (I):

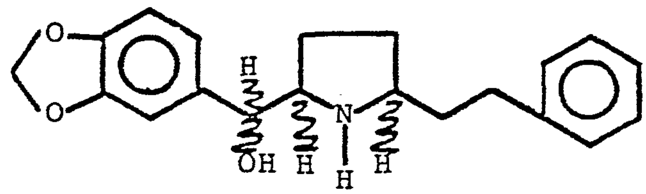

namely, 2-[phenethyl]-5-[(3,4-methylenedioxy)-α-hydroxybenzyl]pyrrolidine, and the pharmaceutically acceptable acid addition salts thereof.

2. A compound according to Claim 1, which is a cis isomer.

3. A compound according to Claim 2 of the formula

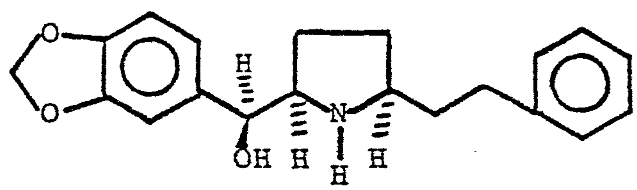

, its enantiomer, and a racemic mixture thereof, namely, (±), (+), and (-) cis erythro 2-[phenethyl]-5-[(3,4-methylenedioxy)-α-hydroxybenzyl]pyrrolidine, and the pharmaceutically acceptable acid addition salts thereof.

4. A compound according to Claim 3, which is ±cis erythro 2-[phenethyl]-5-[(3,4-methylenedioxy)-α-hydroxybenzyl]pyrrolidine, and pharmaceutically acceptable salts thereof.

5. A compound according to Claim 2 of the formula

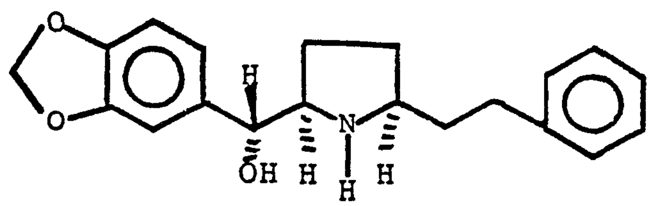

, its enantiomer, and a racemic mixture thereof, namely, (±), (+), and (-) cis threo 2-[phenethyl]-5-[(3,4-methylenedioxy)-α-hydroxybenzyl]pyrrolidine, and the pharmaceutically acceptable acid addition salts thereof.

6. A compound according to Claim 1, which is a trans isomer.

7. A compound according to Claim 6, of the formula

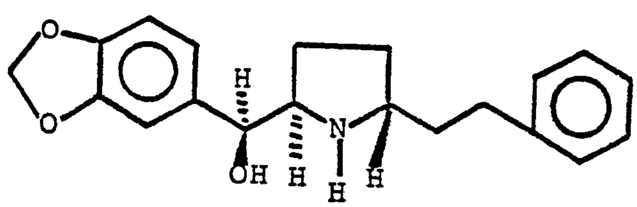

, its enantiomer, and a racemic mixture thereof, namely, (±), (+), and (-) trans erythro 2-[phenethyl]-5-[(3,4-methylenedioxy)-α-hydroxybenzyl]pyrrolidine and the pharmaceutically acceptable acid addition salts thereof.

8. A compound according to Claim 6 of the formula

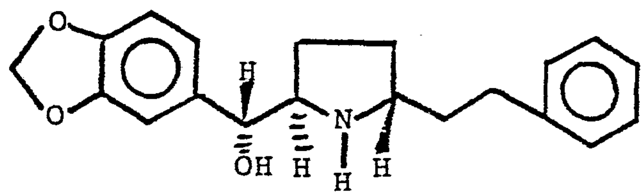

, its enantiomer, and a racemic mixture thereof, namely, (±), (+), and (-) trans threo 2-[phenethyl]-5-[(3,4-methylenedioxy)-α-hydroxybenzyl]pyrrolidine, and the pharmaceutically acceptable acid addition salts thereof.

9. A pharmaceutical composition comprising a compound according to any one of the Claims 1 to 8, in admixture with a pharmaceutically acceptable excipient.

10. A compound of any of Claims 1 to 8 for use in regulating the cardiovascular system in mammals.

11. The use of a compound according to any one of the Claims 1 to 8, in the treatment of medical disorders.

12. A process for preparing a compound according to Claim 1, which process comprises either:

(i) the reduction of a compound of formula $(I)_a$:

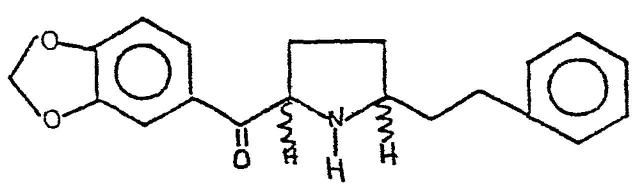

$(I)_a$; or

(ii) the de-protection of a compound of formula $(I)_b$:

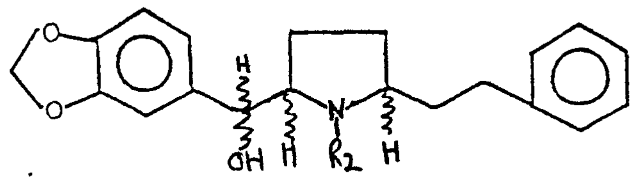

$(I)_b$

wherein $R_2$ is a protecting group; or

(iii) the pyrrolidine forming cyclization of the corresponding epoxy and amino substituted straight chain compound; or

(iv) converting a free base of formula (I) to a salt thereof; or

(v) converting a salt of formula (I) to a free base of formula (I); or

(vi) converting one salt of formula (I) to another salt of formula (I).

13. A compound of formula $(I)_a$ or $(I)_b$ as defined in Claim 12.

CLAIMS

1. A process for preparing a compound of formula (I):

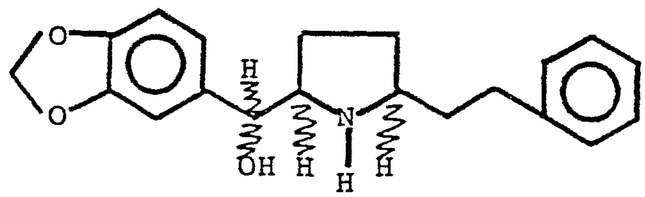

(I)

namely, 2-[phenethyl]-5-[(3,4-methylenedioxy)-α-hydroxybenzyl]pyrrolidine, and the pharmaceutically acceptable acid addition salts thereof, which process comprises either:

(i) the reduction of a compound of formula $(I)_a$:

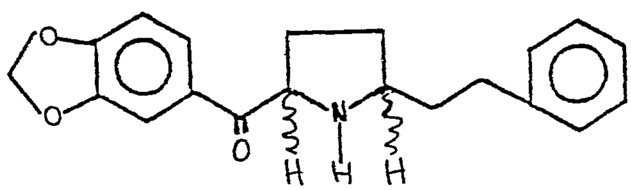

$(I)_a$; or

(ii) the de-protection of a compound of formula $(I)_b$:

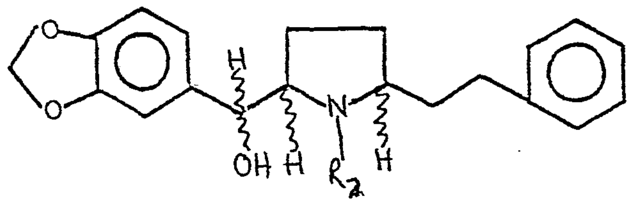

$(I)_b$

wherein $R_2$ is a protecting group; or

(iii) the pyrrolidine forming cyclization of the corresponding epoxy and amino substituted straight chain compound; or

(iv) converting a free base of formula (I) to a salt thereof; or

(v) converting a salt of formula (I) to a free base of formula (I); or

(vi) converting one salt of formula (I) to another salt of formula (I).

2. A process according to Claim 1, for the preparation of a compound which is a cis isomer.

3. A process according to Claim 2 for the preparation of a compound of the formula

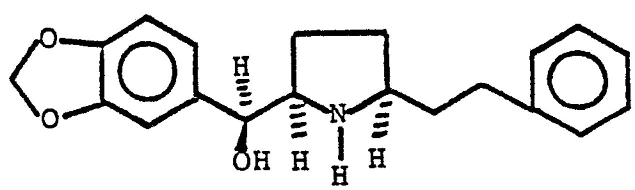

, its enantiomer, and a racemic mixture thereof, namely, (±), (+), and (-) cis erythro 2-[phenethyl]-5-[(3,4-methylenedioxy)-α-hydroxybenzyl]pyrrolidine, and the pharmaceutically acceptable acid addition salts thereof.

4. A process according to Claim 3, for the preparation of ±cis erythro 2-[phenethyl]-5-[(3,4-methylenedioxy)-α-hydroxybenzyl]pyrrolidine, and pharmaceutically acceptable salts thereof.

5. A process according to Claim 2 for the preparation of a compound of the formula

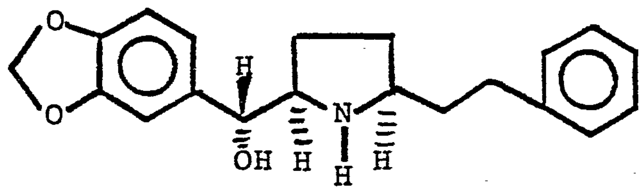

, its enantiomer, and a racemic mixture thereof, namely, (±), (+), and (-) cis threo 2-[phenethyl]-5-[(3,4-methylenedioxy)-α-hydroxybenzyl]pyrrolidine, and the pharmaceutically acceptable acid addition salts thereof.

6. A process according to Claim 1, for the preparation of a compound which is a trans isomer.

7. A process according to Claim 6, for the preparation of a compound of the formula

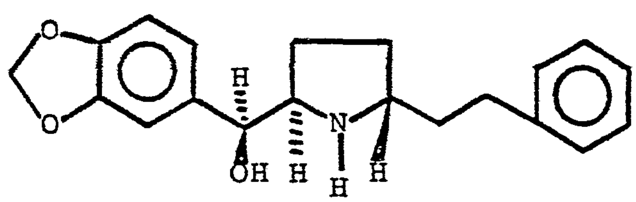

, its enantiomer, and a racemic mixture thereof, namely, (±), (+), and (-) trans erythro 2-[phenethyl]-5-[(3,4-methylenedioxy)-α-hydroxybenzyl]pyrrolidine and the pharmaceutically acceptable acid addition salts thereof.

8. A process according to Claim 6 for the preparation of a compound of the formula

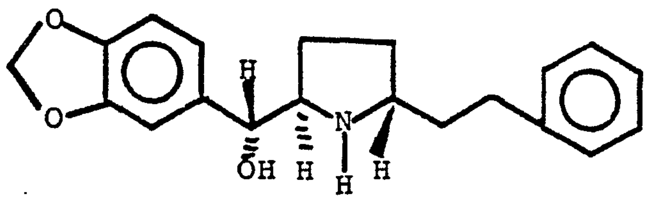

, its enantiomer, and a racemic mixture thereof, namely, (±), (+), and (-) trans threo 2-[phenethyl]-5-[(3,4-methylenedioxy)-α-hydroxybenzyl]pyrrolidine, and the pharmaceutically acceptable acid addition salts thereof.

Process

European Patent Office

# EUROPEAN SEARCH REPORT

0115413
Application number

EP 84 30 0386

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | EP-A-0 050 370 (U.S.V.)<br>* The whole document * | 1-9 | C 07 D 405/06<br>A 61 K 31/40 |
| A | EP-A-0 002 672 (BYK GULDEN L.)<br>* Claims, page 31 * | 1,9 | |
| A | EP-A-0 010 460 (PHARMINDUSTRIE)<br>* Abstract * | 1,9 | |
| A | EP-A-0 022 408 (ROUSSEL-UCLAF)<br>* Abstract * | 1,9 | |
| P,X | EP-A-0 071 399 (SYNTEX INC.)<br>* The whole document * | 1-9 | |

TECHNICAL FIELDS SEARCHED (Int. Cl. 3)

C 07 D 405/00

The present search report has been drawn up for all claims

| Place of search | Date of completion of the search | Examiner |
|---|---|---|
| THE HAGUE | 29-03-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document